# EUROPEAN PATENT SPECIFICATION

(11) **EP 2 633 057 B1**
(45) Date of publication and mention of the grant of the patent: **28.09.2016**
(21) Application number: 11836983.4
(22) Date of filing: 26.10.2011
(51) Int. Cl.: C12N 15/82, A01H 5/00, C07H 21/02

(54) **OVEREXPRESSION OF PLANT MIRNAS FOR PARASITE CONTROL**
ÜBEREXPRESSION VON PFLANZEN-MIRNAS ZUR BEKÄMPFUNG VON PARASITEN
SUREXPRESSION DE MIARN DE PLANTE POUR LUTTER CONTRE LES PARASITES

(30) Priority: 29.10.2010 US 408088 P
(43) Date of publication of application: 04.09.2013
(73) Proprietor: Syngenta Participations AG, 4058 Basel (CH)
(72) Inventor: HUANG, Xiang, Durham North Carolina 27709 (US); SEGUIN, Katie, Durham North Carolina 27709 (US)
(74) Representative: Vossius & Partner Patentanwälte Rechtsanwälte mbB
(86) International application number: PCT/US2011/057813
(87) International publication number: WO 2012/058266

(56) References cited:
- WO-A1-2008/103643
- WO-A1-2012/001626
- WO-A2-2009/133126
- CN-A- 101 544 987
- US-A1- 2007 113 302
- STEEVES RYAN M ET AL: "Transgenic soybeans expressing siRNAs specific to a major sperm protein genesuppress Heterodera glycines reproduction", FUNCTIONAL PLANT BIOLOGY, CSIRO, AU, vol. 33, no. 11, 1 January 2006 (2006-01-01), pages 991-999, XP009091124, ISSN: 1445-4408, DOI: 10.1071/FP06130
- HEWEZI TAREK ET AL: "Arabidopsis Small RNAs and Their Targets During Cyst Nematode Parasitism", MOLECULAR PLANT-MICROBE INTERACTIONS, vol. 21, no. 12, December 2008 (2008-12), pages 1622-1634, XP002720514, ISSN: 0894-0282
- KHAN BAROZAI M Y ET AL: "Identification of micro-RNAs in cotton", PLANT PHYSIOLOGY AND BIOCHEMISTRY, GAUTHIER-VILLARS, PARIS, FR, vol. 46, no. 8-9, 1 August 2008 (2008-08-01), pages 739-751, XP023781412, ISSN: 0981-9428, DOI: 10.1016/J.PLAPHY.2008.05.009 [retrieved on 2008-05-28]
- M. N. ROSSO ET AL: "RNAi and Functional Genomics in Plant Parasitic Nematodes", ANNUAL REVIEW OF PHYTOPATHOLOGY, vol. 47, no. 1, 1 September 2009 (2009-09-01), pages 207-232, XP055020843, ISSN: 0066-4286, DOI: 10.1146/annurev.phyto.112408.132605
- BELLAFIORE S ET AL: "Nematode effectors and plant responses to infection", CURRENT OPINION IN PLANT BIOLOGY, QUADRANT SUBSCRIPTION SERVICES, GB, vol. 13, no. 4, 1 August 2010 (2010-08-01) , pages 442-448, XP027173301, ISSN: 1369-5266 [retrieved on 2010-06-09]

## Description

The invention relates to control of plant parasites and pathogens, particularly methods for controlling nematode parasitism, by overexpression of plant miRNAs.

Plant parasites (pests and pathogens) cause billions of dollars in crop losses worldwide each year. Nematodes, in particular, the soybean cyst nematode, are the number one pests of soybean.

Nematodes are obligate, sedentary endoparasites that feed on the roots, leaves and stems of more than 2,000 vegetables, fruits, and ornamental plants, causing an estimated $100 billion crop loss worldwide. Nematodes are present throughout the United States, but are mostly a problem in warm, humid areas of the south and west, as well as in sandy soils. Soybean cyst nematode (SCN), *Heterodera glycines,* was first discovered in North Carolina in 1954. It is the most serious pest of soybean plants. Once SCN is present in a field, it cannot feasibly be eradicated using known methods. Although soybean is the major economic crop attacked by SCN, SCN parasitizes some fifty hosts in total, including field crops, vegetables, ornamentals, and weeds.

Signs of nematode damage include stunting and yellowing of leaves, as well as wilting of the plants during hot periods. However, nematodes, including SCN, can cause significant yield loss without obvious above-ground symptoms. SCN infection in a plant can 1) result in dwarfed or stunted roots, 2) decrease the number of nitrogen-fixing nodules on the roots, and 3) make the roots more susceptible to attack by other soil-borne plant pests.

SCN has a life cycle consisting of an egg stage, four juvenile stages and an adult stage. After the first molt within the egg, SCN second stage juveniles (J2) hatch, move through the soil, penetrate roots and move toward the vascular cylinder. J2 is the only life stage of the nematode that can infect soybean roots. Migratory juveniles select a host cell in the cortex, endodermis, or pericycle and induce host cell fusion as part of the formation of a permanent feeding site called a syncytium. At this point the nematode becomes sedentary and differentiates to the third (J3) and fourth (J4) juvenile stages and then matures to an adult female or male. The actively feeding nematodes thus steal essential nutrients from the plant resulting in yield loss. As the nematodes feed, they swell and eventually the female nematodes become so large that they break through the root tissue and are exposed on the surface of the root.

Male nematodes, which are not swollen as adults, undergo a metamorphosis to resume a vermiform shape at the J4 stage and migrate back out of the root to fertilize adult females. The males then die, while the females remain attached to the root system and continue to feed. Following fertilization, the female produces eggs, most of which remain inside the body. After dying, the female body develops into a hardened cyst that encases the eggs Cysts eventually dislodge and are found free in the soil. The walls of the cyst become very tough, providing protection for the 200-400 eggs contained within. SCN eggs survive within the cyst until proper hatching conditions occur. Although many of the eggs may hatch within the first year, many will survive within the cysts for several years.

Traditional practices for managing SCN include maintaining proper fertility and soil pH levels in SCN-infested land; controlling other plant diseases, as well as insect and weed pests; using sanitation practices such as plowing, planting, and cultivating of SCN-infested fields only after working non-infested fields; cleaning equipment thoroughly after working in infested fields; not using seed from plants grown on infested land for planting non-infested fields unless the seed has been properly cleaned; rotating infested fields and alternating host crops with non-host crops, such as, corn, oat and alfalfa; using pesticides or fumigants (e.g., nematicides); and planting resistant soybean varieties. While many of these can be effective, in addition to being time consuming and costly to implement, some of these approaches are no longer feasible, such as the application of nematicides, due to their toxicity and negative environmental impact.

WO 2009/133126 A2 discloses double stranded RNA compositions and transgenic plants capable of inhibiting expression of essential genes in parasitic nematodes, and methods associated therewith. Specifically disclosed is the use of RNA interference to inhibit expression of a target essential nematode gene, which is a nematode innexin -like, pas-1, tcp-1, snurportin-1 like, pol delta S, prs-4, rtp-1 or rpn-5 gene, and relates to the generation of plants that have increased resistance to parasitic nematodes.

WO 2008/103643 A1 discloses a non-natural transgenic plant cell expressing at least one invertebrate miRNA in planta for suppression of a target gene of an invertebrate pest including nematode pests. Particularly, the target genes are essential genes including genes that are required for development of the invertebrate pest to a fertile reproductive adult, and which, when silenced or suppressed, result in the death of the invertebrate pest or in the invertebrate pest's inability to successfully reproduce.

US 2007/0113302 discloses mature miRNA sequences and MIR gene sequences from crop plants, including maize and soybean. These MIR genes and their encoded mature miRNAs are stated to be useful, e.g., for modifying developmental pathways, e.g., by affecting cell differentiation or morphogenesis.

Thus, there is currently no efficient and effective approach to control nematode infection in plants. Therefore, there is a need for compositions and methods for preventing, controlling, and reducing nematode parasitism in plants.

Accordingly, the present invention overcomes the deficiencies in the art by providing compositions and methods comprising microRNAs (miRNAs) for control of plant parasites, in particular nematode pests.

The present invention relates in as first embodiment to a method of producing a transgenic plant, plant part, or plant cell having increased resistance to a nematode plant parasite, comprising:
introducing into the plant, plant part, or plant cell, a heterologous miRNA nucleotide sequence, wherein the heterologous miRNA nucleotide sequence comprises a miR164 nucleotide sequence that is at least 75% identical to SEQ ID NO:1, wherein the heterologous miRNA nucleotide sequence is expressed in the plant, plant part, or plant cell, thereby producing a transgenic plant, plant part, or plant cell having increased resistance to a nematode plant parasite relative to a control plant, plant part, or plant cell; and optionally further comprising
regenerating a transgenic plant from the transgenic plant part or plant cell, wherein the regenerated transgenic plant comprises in its genome the heterologous miRNA nucleotide sequence and has increased resistance to the nematode plant parasite relative to a control plant..

In a specific embodiment, the invention relates to the method of embodiment 1, wherein the plant, plant part, or plant cell is a soybean plant, soybean plant part, or soybean plant cell.

In a further specific embodiment, the invention relates to the method of embodiment 2, wherein the nematode plant parasite is a soybean cyst nematode.

In another specific embodiment, the transgenic soybean plant, soybean plant part, or soybean plant cell has reduced soybean cyst nematode cyst formation relative to a control soybean plant, soybean plant part, or soybean plant cell.

In certain embodiments, the present invention relates to the method of any of the preceding embodiments, further comprising regenerating a transgenic soybean plant from the transgenic soybean plant part or plant cell, wherein the regenerated transgenic soybean plant comprises in its genome the heterologous miRNA nucleotide sequence and has increased resistance to the nematode plant parasite relative to a control soybean plant, and/or part thereof and/or has reduced soybean cyst nematode cyst formation relative to a control soybean plant, and/or part thereof.

Any reference to the invention or to aspects or embodiments of the invention mentioned herein, which exceed the scope of the invention as represented by the claims do not form part of the invention but represent background information only useful for understanding the invention.

In certain other embodiments, the present invention relates to the method of any of the preceding embodiments, further comprising obtaining a progeny soybean plant derived from the transgenic soybean plant, wherein said progeny plant comprises in its genome the heterologous miRNA nucleotide sequence and has increased resistance to nematode parasitism and/or reduced soybean cyst nematode cyst formation relative to a control soybean plant.

The invention further relates to the method of any one of the preceding embodiments, wherein the heterologous miR164 nucleotide sequence is present in a nucleic acid construct, particularly wherein the heterologous miR164 nucleotide sequence is operably associated with a promoter.

In various embodiments, the invention relates to the method of any of the preceding embodiments, wherein the introducing is via bacterial-mediated transformation, particle bombardment transformation, calcium-phosphate-mediated transformation, cyclodextrin-mediated transformation, electroporation, liposome-mediated transformation, nanoparticle-mediated transformation, polymer-mediated transformation, virus-mediated nucleic acid delivery, whisker-mediated nucleic acid delivery, microinjection, sonication, infiltration, polyethyleneglycol-mediated transformation, any other electrical, chemical, physical or biological mechanism that results in the introduction of nucleic acid into the plant, plant part or cell thereof, or a combination thereof.

The invention further provides in a further embodiment a soybean plant, soybean plant part or soybean plant cell having increased resistance to nematode parasitism, soybean cyst nematode parasitism, and/or reduced formation of soybean cyst nematode cysts, wherein said soybean plant, soybean plant part or soybean plant cell comprises a heterologous miRNA nucleotide sequence comprising a miR164 nucleotide sequence that is at least 75% identical to SEQ ID NO:1, wherein the heterologous miRNA nucleotide sequence is expressed in the soybean plant, soybean plant part, or soybean plant cell, thereby producing a transgenic soybean plant, soybean plant part, or soybean plant cell having increased resistance to a nematode plant parasite relative to a control plant, plant part, or plant cell.

Also comprised within the scope of the present invention is a soybean seed produced by the soybean plant of the preceding embodiment, wherein the seed comprises in its genome the heterologous miRNA nucleotide sequence as defined in the preceding embodiment, particularly the nucleotide sequence of SEQ ID NO:1.

In one embodiment, the present invention provides a soybean seed meal produced from the soybean seed of any one of the preceding embodiments relating to soybean seed, wherein the seed meal comprises the heterologous miRNA nucleotide sequence.

The subject matter of the invention, for which protection is sought, is defined in the claims. Any reference to the invention or to aspects or embodiments of the invention mentioned herein, which exceed the scope of the invention as represented by the claims does not form part of the invention but represent background information only useful for understanding the invention.

One aspect of the invention is a method of producing a transgenic plant, plant part, or plant cell having increased resistance to a plant parasite, comprising introducing into the plant, plant part, or plant cell, a heterologous microRNA164 (miR164) nucleotide sequence, wherein the heterologous miR164 nucleotide sequence is expressed in the plant, plant part or plant cell, thereby producing a transgenic plant, plant part, or cell having increased resistance to a plant parasite relative to a control plant, plant part, or plant cell.

Another aspect of the present invention provides a method of producing a transgenic soybean plant, soybean plant part, and/or soybean plant cell having increased resistance to soybean cyst nematode parasitism, comprising introducing into the soybean plant, soybean plant part, and/or soybean plant cell, a heterologous microRNA164 (miR164) nucleotide sequence, wherein the heterologous miR164 nucleotide sequence is expressed in the plant, plant part and/or plant cell, thereby producing a transgenic soybean plant, soybean plant part, and/or soybean plant cell having increased resistance to soybean cyst nematode parasitism relative to a control soybean plant, soybean plant part, and/or soybean plant cell.

A further aspect of the present invention provides a method of increasing resistance to soybean cyst nematode parasitism in a soybean plant, or part thereof, comprising: a) introducing into a soybean plant cell a heterologous miR164 nucleotide sequence, wherein the heterologous miR164 nucleotide sequence is expressed in the soybean plant cell; and b) regenerating a transgenic soybean plant, or part thereof, from the soybean plant cell of (a), wherein the transgenic soybean plant, or part thereof, comprises in its genome the heterologous miR164 nucleotide sequence and has increased resistance to soybean cyst nematode parasitism relative to a control soybean plant, soybean plant part, or soybean plant cell.

Another aspect of the invention provides a method of reducing soybean cyst nematode cyst formation in a soybean plant, soybean plant part, and/or soybean plant cell, comprising: introducing into the soybean plant, soybean plant part, and/or soybean plant cell a heterologous miR164 nucleotide sequence, wherein the heterologous miR164 nucleotide sequence is expressed in the soybean plant, soybean plant part, and/or soybean plant cell, thereby reducing soybean cyst nematode cyst formation in a soybean plant, soybean plant part, and/or soybean plant cell relative to a control soybean plant, soybean plant part, and/or soybean plant cell.

In an additional aspect, the present invention provides a method of reducing soybean cyst nematode cyst formation in a soybean plant, or part thereof, comprising a) introducing into a soybean plant cell a heterologous miR164 nucleotide sequence, wherein the heterologous miR164 nucleotide sequence is expressed in the plant cell; and b) regenerating a transgenic soybean plant, or part thereof, from the soybean plant cell of (a), wherein the transgenic soybean plant, or part thereof, comprises in its genome the heterologous miR164 nucleotide sequence and has reduced formation of soybean cyst nematode cysts relative to a control soybean plant, soybean plant part, or soybean plant cell.

Additional aspects of the invention provide compositions including nucleic acid constructs for transforming a plant, plant part and/or plant cell, as well as the transformed plant, plant parts and/or plant cells thereof.

These and other aspects of the invention are set forth in more detail in the description of the invention below.
**Fig. 1** shows a soybean gma-MIR164 sequence (precursor sequence). The mature miRNA gma-miR164 sequence processed from the precursor is underlined.
**Figs. 2A-B** show the expression vectors 15312 prCMP-miR164-tNOS **(****Fig. 2A****)** and B-15312-prAR6-cGUS-tNOS **(****Fig. 2B****).** prCMP is the cestrum virus promoter *(See,* e.g, US Patent No. 7,166,770); prAR6 is an *Arabidopsis* root promoter *(See,* e.g., US Patent No. 7,615,624); tNOS is the terminator from the *nos* gene of *Agrobacterium tumefaciens,* and cGUS is a beta-glucuronidase reporter gene.
**Fig. 3** is a graph of the data showing the effects of overexpression of gma-miR164 on soybean cyst nematode (SCN) cyst formation.
**Fig. 4** is a graph showing the effects of overexpression of miR396b on cyst development on transgenic hairy roots.

This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. In addition, numerous variations and additions to the various embodiments suggested herein will be apparent to those skilled in the art in light of the instant disclosure, which do not depart from the instant invention. Hence, the following descriptions are intended to illustrate some particular embodiments of the invention, and not to exhaustively specify all permutations, combinations and variations thereof.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

All publications, patent applications, patents, and other references mentioned herein are incorporated by reference in their entirety

As used herein, "a," "an" or "the" can mean one or more than one. For example, a cell can mean a single cell or a multiplicity of cells.

As used herein, "and/or" refers to and encompasses any and all possible combinations of one or more of the associated listed items, as well as the lack of combinations when interpreted in the alternative (or).

Further, the term "about," as used herein when referring to a measurable value such as an amount of a compound or agent, dose, time, temperature, and the like, is meant to encompass variations of ± 20%, ± 10%, ± 5%, ± 1%, ± 0.5%, or seven ± 0.1% of the specified amount.

As used herein, the transitional phrase "consisting essentially of' means that the scope of a claim is to be interpreted to encompass the specified materials or steps recited in the claim and those that do not materially affect the basic and novel characteristic(s) of the claimed invention. Thus, the term "consisting essentially of" when used in a claim of this invention is not intended to be interpreted to be equivalent to "comprising."

It will be understood that, although the terms "first," "second," etc. may be used herein to describe various elements, these elements should not be limited by these terms. These terms are only used to distinguish one element from another. Thus, a "first" element (e.g., a first promoter sequence) as described herein could also be termed a "second" element (e.g., a second promoter sequence) without departing from the teachings of the present invention.

MicroRNAs (miRNAs) are non-protein coding RNAs, generally of between about 19 to about 25 nucleotides (commonly about 20-24 nucleotides in plants). miRNAs direct cleavage in *trans* of target transcripts, regulating the expression of genes involved in various regulation and development pathways (Bartel, Cell, 116:281-297 (2004); Zhang et al. Dev. Biol. 289:3-16 (2006)). miRNAs have been shown to be involved in different aspects of plant growth and development as well as in signal transduction and protein degradation. In addition, growing evidence indicates that small endogenous mRNAs including miRNAs may also be involved in biotic stress responses such as parasite attack. Since the first miRNAs were discovered in plants (Reinhart et al. Genes Dev. 16:1616-1626 (2002), Park et al. Curr. Biol. 12:1484-1495 (2002)), many hundreds have been identified. Further, many plant miRNAs have been shown to be highly conserved across very divergent taxa. (Floyd et al. Nature 428:485-486 (2004); Zhang et al. Plant J. 46:243-259 (2006)). Many microRNA genes (MIR genes) have been identified and made publicly available in a database ("miRBase," available on line at microrna.sanger.ac.uk/sequences). miRNAs are also described in U.S. Patent Publications 2005/0120415 and 2005/144669A1.

Genes encoding miRNAs yield primary miRNAs ("pri-miRNA") of 70 to 300 bp in length that can form imperfect stem-loop structures. A single pri-miRNA may contain from one to several miRNA precursors. In animals, pri-miRNAs are processed in the nucleus into shorter hairpin RNAs of about 65 nucleotides (referred to as precursor miRNAs (pre-miRNAs)) by the RNaseIII enzyme Drosha and its cofactor DGCR8/Pasha. The pre-miRNA is then exported to the cytoplasm, where it is further processed by another RNaseIII enzyme, Dicer, releasing a miRNA/miRNA* duplex of about 22 nt in size. In contrast to animals, in plants, the processing of pri-miRNAs into mature miRNAs occurs entirely in the nucleus using a single RNaseIII enzyme, DCL1 (Dicer-like 1). (Zhu. Proc. Natl. Acad. Sci. 105:9851-9852 (2008)). Many reviews on microRNA biogenesis and function are available, for example, *see,* Bartel. Cell 116:281-297 (2004), Murchison et al. Curr. Opin. Cell Biol. 16:223-229 (2004), Dugas et al. Curr. Opin. Plant Biol. 7:512-520 (2004) and Kim. Nature Rev. Mol. Cell Biol. 6:376-385 (2005).

The present invention is directed to the discovery that overexpression in a plant of particular heterologous plant miRNAs can result in increased resistance to parasites such as the soybean cyst nematode (SCN).

Thus, in some embodiments, the present invention provides a method of producing a transgenic plant, plant part, and/or plant cell having increased resistance to a plant parasite, comprising introducing into the plant, plant part, and/or plant cell, a heterologous miRNA nucleotide sequence, wherein the heterologous miRNA nucleotide sequence is expressed in the plant, plant part and/or plant cell, thereby producing a transgenic plant, plant part, and/or plant cell having increased resistance to the plant parasite relative to a control plant, plant part, and/or plant cell that is not transformed with the heterologous miRNA nucleotide sequence. In some embodiments, the plant is a soybean plant.

In some embodiments, the heterologous miRNA nucleotide sequence is a plant miRNA. Nonlimiting examples include any family members of the following plant miRNAs: miR156, miR159, miR160, miR161, miR162, miR163, miR164, miR165, miR166, miR167, miR168, miR169, miR170, miR171, miR172, miR173, miR319, miR390, miR393, miR395, miR396, miR397, miR398, miR399, miR408, and/or miR447.

miRNAs of the present invention additionally include miRNAs identified in soybean roots. Thus, nonlimiting examples of soybean root miRNAs include gma-MIR1507a (MI0007219), gma-MIR1507b (MI0010573), gma-MIR1508a (MI0007220), gma-MIR1508b (MI0010574), gma-MIR1509a (MI0007221), gma-MIR1509b (MI0010730), gma-MIR1510a (MI0007222), gma-MIR1510b (MI0010575), gma-MIR1511 (MI0007223), gma-MIR1512 (MI0007224), gma-MIR1513 (MI0007225), gma-MIR1514a (MI0007226), gma-MIR1514b (MI0007227), gma-MIR1515 (MI0007228), gma-MIR1516 (MI0007229), gma-MIR1517 (MI0007230), gma-MIR1518 (MI0007231), gma-MIR1519 (MI0007232), gma- MIR 520d (MI0007233), gma-MIR1520a (MI0007235), gma-MIR1520b (MI0007243), gma-MIR1520c (MI0007252), gma-MIR1520e (MI0016484), gma-MIR1520f (MI0016485), gma-MIR1520g (MI0016486), gma-MIR1520h (MI0016496), gma-MIR1520i (MI0016498), gma-MIR1520j (MI0016504), gma-MIR15201c (MI0016532), gma-MIR15201 (MI0016533), gma-MIR1520m (MI0016534), gma-MIR1520n (MI0016536), gma-MIR1520o (MI0016537), gma-MIR1520r (MI0016554), gma-MIR1520p (MI0016563), gma-MIR1520q (MI0016573), gma-MIR1521 (MI0007236), gma-MIR1522 (MI0007237), gma-MIR1523 (MI0007240), gma-MIR1524 (MI0007241), gma-MIR1525 (MI0007242), gma-MIR1526 (MI0007244), gma-MIR1527 (MI0007245), gma-MIR1528 (MI0007246), gma-MIR1529 (MI0007248), MIR1530 (MI0007249), gma-MIR1531 (MI0007250), gma-MIR1532 (MI0007251), gma-MIR1533 (MI0007253), gma-MIR1534 (MI0007254), gma-MIR1535 (MI0007257), gma-MIR1536 (MI0007234), gma-MIR171b (MI0007217), gma-MIR482a (MI0007218), gma-MIR482b (MI0016527), gma-MIR845, gma-MIR894, gma-MIR1055, gma-MIR156dMI0001770), gma-MIR156e (M0001771), gma-MIR156c (MI0001772), gma-MIR156a (MI0001784), gma-MIR156b (MI0001790), gma-MIR156f (MI0016559), gma-MIR156g (MI0016580), gma-miR159a (MI0001773), gma-miR159b (MI0007206), gma-miR159c (MI0007207), gma-miR159d (MI0016581), gma-miR160 (MI0001774), gma-miR162 (MI0007208), gma-miR164 (MI0007209), gma-miR166a (MI0001775), gma-miR166b (MI0001776), gma-MIR167aMI0001777), gma-MIR167b (MI0001778), gma-MIR167c (MI0007210), gma-MIR167d (MI0010571), gma-MIR167e (MI0010725), gma-MIR167f (MI0010726), gma-MIR167g (MI0016548), gma-miR168 (MI0001779), gma-MIR169a (MI0001791), gma-MIR169b (MI0007211), gma-MIR169c (MI0007212), gma-MIR169d (MI0016571), gma-MIR169e (MI0016576), gma-miR171a (MI0007213), gma-miR171b (MI0007217), gma-miR171c (MI0016575), gma-MIR172a (MI0001780), gma-MIR172b (MI0001781), gma-MIR172c (MI0010727), gma-MIR172d (MI0010728), gma-MIR172e (MI0010729), gma-MIR172f (MI0016574), gma-miR319a (MI0001782), gma-miR319b (MI0001783), gma-miR319c (MI0001789), gma-miR390a (MI0007214), gma-miR390b (MI0007215), gma-miR393 (MI0007216), gma-MIR396a (MI0001785), gma-MIR396b (MI0001786), gma-MIR396c (MI0010572), gma-MIR396d (MI0016503), gma-MIR396e (MI0016586), and/or gma-miR397.

miRNAs of the present invention also include the members of the miRNA164 family. Nonlimiting examples of miRNA164 family members include aly-MIR164a (MI0014527), aly-MIR164b (MI0014528), aly-MIR164c (MI0014529), ath-MIR164aMI0000197), ath-MIR164b (MI0000198), ath-MIR164c (MI0001087), bna-MIR164 (MI0006478), bra-MIR164a (MI0010651), csi-MIR164 (MI0013295), ctr-MIR164 (MI0013306), far-MIR164a(MI0016611), far-MIR164b(MI0016617), ghr-MIR164 (MI0013538), gma-MIR164 (MI0007209), mtr-MIR164a (MI0005573), mtr-MIR164b (MI0005612), mtr-MIR164c (MI0005616), mtr-MIR164d (MI0005617), osa-MIR164a (MI0000668), osa-MIR164b (MI0000669), osa-MIR164c (MI0001103), osa-MIR164d (MI0001104), osa-MIR164e (MI0001105), osa-MIR164f (MI0001159), ptc-MIR164a (MI0002212), ptc-MIR164b (MI0002213), ptc-MIR164c (MI0002214), ptc-MIR164d (MI0002215), ptc-MIR164e (MI0002216), ptc-MIR164f (MI0002217), rco-MIR164a (MI0013382), rco-MIR164b (MI0013383), rco-MIR164c (MI0013384), rco-MIR164d (MI0013385), sbi-MIR164a (MI0001512), sbi-MIR164b (MI0001549), sbi-MIR164c (MI0001852), sbi-MIR164d (MI0010865), sbi-MIR164e (MI0010866), tae-MIR164 (MI0006173), vvi-MIR164a (MI0006503), vvi-MIR164b (MI0006504), vvi-MIR164c (MI0006505), vvi-MIR164d (MI0006506), zma-MIR164a (MI0001469), zma-MIR164b (MI0001471), zma-MIR164c (MI0001472), zma-MIR164d (MI0001470), zma-MIR164a (MI0001469), zma-MIR164b (MI0001471),zma-MIR164c (MI0001472), zma-MIR164d (MI0001470),zma-MIR164e (MI0013193), zma-MIR164f (MI0013194), zma-MIR164g (MI0013195), and/or zma-MIR164h (MI0013196).

In other embodiments, the present invention provides a method of producing a transgenic plant, plant part, and/or plant cell having increased resistance to a plant parasite, comprising introducing into the plant, plant part, and/or plant cell, a heterologous miR164 nucleotide sequence, wherein the heterologous miR164 nucleotide sequence is expressed in the plant, plant part and/or plant cell, thereby producing a transgenic plant, plant part, and/or plant cell having increased resistance to the parasite relative to a control plant, plant part, and/or plant cell that is not transformed with the heterologous miR164 nucleotide sequence.

As used herein, "plant" means any plant and thus includes, for example, angiosperms, gymnosperms, bryophytes, ferns and/or fern allies. Non-limiting examples of plants of the present invention include soybean, beans in general, corn, cereal crops including but not limited to barley, ryes, oats, wheat, and the like, *Brassica* spp., clover, cocoa, coffee, cotton, flax, maize, millet, peanut, rape/canola, rice, rye, safflower, sorghum, sugarcane, sugar beet, sunflower, sweet potato, tea, vegetables including but not limited to broccoli, brussel sprouts, cabbage, carrot, cassava, cauliflower, cucurbits, lentils, lettuce, pea, peppers, potato, radish and tomato, grasses, fruits including, but not limited to, apples, pears, peaches apricots and citrus, avocado, banana, coconuts, pineapple and walnuts; and flowers including, but not limited to, carnations, orchids, roses, and any combination thereof.

Additional examples of plants of the present invention are any plant species or plant varieties susceptible to soybean cyst nematode infection including, but not limited to, China pinks, edible beans, lespedeza, vetch (common, hairy or winter), lupine, clover (crimson, scarlet or alsike), sweetclover, birdsfoot trefoil, crownvetch, garden pea, cowpea, black-eyed pea, soybeans (wild and cultivated), black locust, honey locust, portulaca, Bells of Ireland, common chickweed, mousear chickweed, mullein, sicklepod, *Digitalis penstemon,* pokeweed, purslane, bittercress, Rocky Mountain beeplant, spotted geranium, toadflax, winged pigweed, *Psoralea spp., Cleome serrulata,* vetch (American, Carolina or wood), burclover *(Medicago minima),* chick-weed (*Cerastium vulgatum*), dalea, Canadian milkvetch, hemp sesbania, borage, canary bird flower, cup flower, caraway, Chinese lantern plant, blue gem viscaria, coralbell, Margaret double carnation, *Rosa multiflora,* pink queen, geranium (*Geranium maculatum*), cup-flower, delphinium, foxglove, geum, common horehound, poppy, sage, snapdragon, beard-tongue (*Penstemon digitalis*), *Desmodium nudifolorum, D. marilandicum, D. viridiflorum,* corn cockle, sweet basil, sweetpea, verbena, henbit (*Lamium amplexicaule*), purple deadnettle (*Lamium purpureum*), (field pennycress (*Thlaspi arvense*), shepherd's-purse (*Capsella bursa-pastoris*), hop clovers, beggars weed, tick clover, corn cockle, hogpeanut, milkpea, and wildbean (*Strophostyles helvola*).

As used herein, the term "plant part" includes but is not limited to embryos, pollen, ovules, seeds, leaves, flowers, branches, fruit, kernels, ears, cobs, husks, stalks, roots, root tips, anthers, plant cells including plant cells that are intact in plants and/or parts of plants, plant protoplasts, plant tissues, plant cell tissue cultures, plant calli, plant clumps, and the like. Further, as used herein, "plant cell" refers to a structural and physiological unit of the plant, which comprises a cell wall and also may refer to a protoplast. A plant cell of the present invention can be in the form of an isolated single cell or can be a cultured cell or can be a part of a higher-organized unit such as, for example, a plant tissue or a plant organ.

The term "parasite" as used herein includes but is not limited to nematode plant pests, insect plant pests and fungal and bacterial plant pathogens. Non-limiting examples of nematode pests include cyst nematodes (*Heterodera* spp.), especially the soybean cyst nematode (*Heterodera glycines*), root knot nematodes (*Meloidogyne* spp.), lance nematodes (*Hoplolaimus* spp.), stunt nematodes (*Tylenchorhynchus* spp.), spiral nematodes (*Helicotylenchus* spp.), lesion nematodes (*Pratylenchus* spp.), sting nematodes (*Belonoluimus* spp.), reniform nematodes (*Rotylenchulus reniformis*), burrowing nematodes (*Radopholus similis*) and ring nematodes (*Criconema* spp.).

Non-limiting examples of fungal plant pathogens include Asian rust (*Phakospora pachyrhizi*), soybean sudden death (*Fusarium solani*), white mold (*Sclertinia sclerotiorum*), brown stem rot (*Phialophora gregata*)*,* root and stem rot (*Phytophthora sojae*), and corn common rust (*Puccinia sorghi*)*.* Other fungal pathogens include, but are not limited to, *Alubgo* spp., *Phytophthora* spp., *Pythium* spp., *Aphanomyces* spp., *Peronospora* spp..

Non-limiting examples of insect plant pests include Lepidopteran insects including *Ostrinia nubilalis* (European corn borer), *Plutella xylostella* (diamondback moth), *Spodoptera frugiperda* (fall armyworm), *Agrotis ipsilon* (black cutworm), *Helicoverpa zea* (corn earworm), *Heliothis virescens* (tobacco budworm), *Spodoptera exigua* (beet armyworm), *Diatraea grandiosella* (southwestern corn borer), *Diatraea saccharalis* (sugarcane borer), *Sesamia nonagroides* (mediterranean corn borer), *Helicoverpa punctigera* (native budworm) and *Helicoverpa armigera* (cotton bollworm); and Coleopteran insects including corn rootworm *Diabrtoica* spp., wireworm (*Melanotus* spp.), white wireworm (*Aeolus* spp.), maize billbug (*Sphenophorus callus*), and white grubs (*Phyllophaga* spp.).

Thus, in some embodiments, the present invention provides a method of producing a transgenic soybean plant, soybean plant part, and/or soybean plant cell having increased resistance to a plant parasite, comprising introducing into the soybean plant, soybean plant part, and/or soybean plant cell, a heterologous miRNA nucleotide sequence, wherein the heterologous miRNA nucleotide sequence is expressed in the soybean plant, soybean plant part and/or soybean plant cell, thereby producing a soybean transgenic plant, soybean plant part, and/or soybean plant cell having increased resistance to the parasite relative to a control soybean plant, soybean plant part, and/or soybean plant cell that is not transformed with the heterologous miRNA nucleotide sequence.

Thus, in some embodiments, the present invention provides a method of producing a transgenic soybean plant, soybean plant part, and/or soybean plant cell having increased resistance to soybean cyst nematode parasitism, comprising introducing into the soybean plant, soybean plant part, and/or soybean plant cell, a heterologous miR164 nucleotide sequence, wherein the heterologous miR164 nucleotide sequence is expressed in the plant, plant part and/or plant cell, thereby producing a transgenic soybean plant, soybean plant part, and/or soybean plant cell having increased resistance to soybean cyst nematode parasitism relative to a control soybean plant, soybean plant part, and/or soybean plant cell that is not transformed with the heterologous miR164 nucleotide sequence. A non-limiting example of a heterologous miR164 nucleotide sequence is the nucleotide sequence of **SEQ ID. NO:1.** Thus, in some embodiments of the present invention, a method of producing a transgenic soybean plant, soybean plant part, and/or soybean plant cell having increased resistance to soybean cyst nematode parasitism is provided, the method comprising introducing into the soybean plant, soybean plant part, and/or soybean plant cell, a heterologous nucleotide sequence encoding the nucleotide sequence of **SEQ ID NO:1,** wherein the heterologous nucleotide sequence is expressed in the plant, plant part and/or plant cell, thereby producing a transgenic soybean plant, soybean plant part, and/or soybean plant cell having increased resistance to soybean cyst nematode parasitism relative to a control soybean plant, soybean plant part, and/or soybean plant cell that is not transformed with the heterologous nucleotide sequence encoding the nucleotide sequence of **SEQ ID NO:1.**

As used herein, "heterologous" refers to a nucleotide sequence that either originates from another species or is from the same species or organism but is modified from either its original form or the form primarily expressed in the cell. Thus, a nucleotide sequence derived from an organism or species different from that of the cell into which the nucleotide sequence is introduced, is heterologous with respect to that cell and the cell's descendants. In addition, a heterologous nucleotide sequence includes a nucleotide sequence derived from and inserted into the same natural, original cell type, but which is present in a non-natural state, e.g. present in a different copy number, and/or under the control of different regulatory sequences than that found naturally in nature.

The terms "increase," "increased," "enhance," "enhanced," "enhancing," and "enhancement" (and grammatical variations thereof), as used herein, describe an increase in the resistance of a plant to a parasite (e.g., a soybean plant having increased resistance to the soybean cyst nematode) by the introduction of a heterologous miRNA nucleotide sequence of the present invention into the plant, thereby producing a transgenic plant having increased resistance to the parasite. This increase can be observed by comparing the resistance of the plant transformed with the heterologous miRNA nucleotide sequence of the invention to a plant (e.g., soybean) that is not transformed with the heterologous miRNA nucleotide sequence of the invention (e.g., a soybean plant transformed with the heterologous miR164 nucleotide sequence compared to a soybean plant that is not transformed with the heterolgous miR164 nucleotide sequence).

In some embodiments of the present invention, a heterologous miRNA nucleotide sequence is overexpressed in the plant, plant part and/or plant cell, thereby producing a transgenic plant, plant part, and/or plant cell having increased resistance to a parasite relative to a control plant, plant part, and/or plant cell that is not transformed with the heterologous miRNA nucleotide sequence. In some embodiments of the present invention, the heterologous miRNA is miR164. In still other embodiments, the miRNA is gma-miR164.

Thus, in some embodiments of the present invention, the heterologous miR164 nucleotide sequence is overexpressed in the plant, plant part and/or plant cell, thereby producing a transgenic plant, plant part, and/or plant cell having increased resistance to a parasite relative to a control plant, plant part, and/or plant cell that is not transformed with the heterologous miR164 nucleotide sequence.

Accordingly in further embodiments of the present invention, the heterologous miR164 nucleotide sequence is overexpressed in the soybean plant, soybean plant part, and/or soybean plant cell, thereby producing a transgenic soybean plant, soybean plant part, and/or soybean plant cell having increased resistance to soybean cyst nematode parasitism relative to a control soybean plant, soybean plant part, and/or soybean plant cell that is not transformed with the heterologous miR164 nucleotide sequence.

The terms "overexpressed," "overexpression" and grammatical variations thereof as used herein refer to a level of expression which is greater than normal physiological levels. Thus, a plant, plant part and/or plant cell transformed with a nucleotide sequence of the present invention (e.g., a construct comprising a miRNA nucleotide sequence (e.g., miRNA164)) expresses the product of the construct at a level greater than that expressed by a plant, plant part and/or plant cell that is not transformed with the nucleotide sequence of the present invention. Overexpression can be compared or determined at any developmental and/or temporal stage.

Accordingly, overexpression can take place in a plant, plant part and/or plant cell normally lacking expression of a nucleic acid molecule or a nucleotide sequence functionally equivalent or identical to a nucleic acid molecule or nucleotide sequence of the present invention (e.g., miR164). Overexpression can also occur in a plant, plant part and/or plant cell where endogenous expression of the nucleic acid molecule or nucleotide sequence of the present invention or functionally equivalent nucleic acid molecule or nucleotide sequence normally occurs, but such normal expression is at a lower level than the expression of a nucleic acid molecule or nucleotide sequence of the invention. Overexpression thus results in a greater than normal production, or "overproduction" of the product of the nucleic acid molecule or nucleotide sequence in the plant, plant part and/or plant cell.

The overexpression may be constitutive, or alternatively, the nucleic acid(s) of the present invention may be expressed in a tissue-specific manner and/or from an inducible promoter including a promoter which is responsive to external stimuli, such as chemical application, and/or to parasite infection. Thus, overexpression may occur throughout a plant, in specific tissues of the plant, and/or in the presence or absence of particular environmental signals, depending on the promoter used. In one embodiment of the invention, the promoter employed can be one that is rapidly and transiently and/or highly transcribed after parasite infection. In some particular embodiments, the promoter is a root-specific promoter. Promoters functional in plants are well known in the art, as described herein.

As used herein, the term "nucleic acid," "nucleic acid molecule," and/or "nucleotide sequence" refers to a heteropolymer of nucleotides or the sequence of these nucleotides from the 5' to 3' end of a nucleic acid molecule and includes DNA or RNA molecules, including cDNA, a DNA fragment, genomic DNA, synthetic (*e.g*., chemically synthesized) DNA, plasmid DNA, mRNA, and anti-sense RNA, any of which can be single stranded or double stranded. The terms "nucleotide sequence" "nucleic acid," "nucleic acid molecule," "oligonucleotide" and "polynucleotide" are also used interchangeably herein to refer to a heteropolymer of nucleotides. Nucleic acid sequences provided herein are presented herein in the 5' to 3' direction, from left to right and are represented using the standard code for representing the nucleotide characters as set forth in the sequence rules for the U.S. Patent and Trademark Office, 37 CFR §§1.821 - 1.825, and the World Intellectual Property Organization (WIPO) Standard ST.25.

As used herein, the term "gene" refers to a nucleic acid molecule capable of being used to produce mRNA, antisense RNA, miRNA, and the like. Genes may or may not be capable of being used to produce a functional protein or gene product. Genes can include both coding and non-coding regions (e.g., introns, regulatory elements, promoters, enhancers, termination sequences and/or 5' and 3' untranslated regions). A gene may be "isolated" by which is meant a nucleic acid that is substantially or essentially free from components normally found in association with the nucleic acid in its natural state. Such components include other cellular material, culture medium from recombinant production, and/or various chemicals used in chemically synthesizing the nucleic acid.

The terms "complementary" or "complementarity," as used herein, refer to the natural binding of polynucleotides under permissive salt and temperature conditions by base-pairing. For example, the sequence "A-G-T" binds to the complementary sequence "T-C-A." Complementarity between two single-stranded molecules may be "partial," in which only some of the nucleotides bind, or it may be complete when total complementarity exists between the single stranded molecules. The degree of complementarity between nucleic acid strands has significant effects on the efficiency and strength of hybridization between nucleic acid strands.

The term "nucleic acid fragment" will be understood to mean a nucleotide sequence of reduced length relative to a reference nucleic acid or nucleotide sequence and comprising, consisting essentially of and/or consisting of a nucleotide sequence of contiguous nucleotides identical or almost identical (e.g., 80%, 81%, 82%, 83%, 84%, 85%, 86%, 87%, 88%, 89%, 90%, 91%, 92%, 93%, 94%, 95%, 96%, 98%, 99% identical) to the reference nucleic acid or nucleotide sequence. Such a nucleic acid fragment according to the invention may be, where appropriate, included in a larger polynucleotide of which it is a constituent. In some embodiments, such fragments can comprise, consist essentially of and/or consist of, oligonucleotides having a length of at least about 6, 7, 8, 9, 10, 11, 12, 13, 14, 15, 16, 17, 18, 19, 20, 21, 22, 23, 24, 25, 50, 75, 100, 150, 200, 250, 300, 350, 400, 450, 500, 750, or 1000 consecutive nucleotides of a nucleic acid or nucleotide sequence according to the invention.

An "isolated" nucleic acid of the present invention is generally free of nucleic acid sequences that flank the nucleic acid of interest in the genomic DNA of the organism from which the nucleic acid was derived (such as coding sequences present at the 5' or 3' ends). However, the nucleic acid of this invention can include some additional bases or moieties that do not deleteriously affect the basic structural and/or functional characteristics of the nucleic acid. "Isolated" does not mean that the preparation is technically pure (homogeneous).

Thus, an "isolated nucleic acid" is present in a form or setting that is different from that in which it is found in nature and is not immediately contiguous with nucleotide sequences with which it is immediately contiguous (one on the 5' end and one on the 3' end) in the naturally occurring genome of the organism from which it is derived. Accordingly, in one embodiment, an isolated nucleic acid includes some or all of the 5' non-coding (e.g., promoter) sequences that are immediately contiguous to a coding sequence. The term therefore includes, for example, a recombinant nucleic acid that is incorporated into a vector, into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment), independent of other sequences. Thus, a nucleic acid found in nature that is removed from its native environment and transformed into a plant is still considered "isolated" even when incorporated into the genome of the resulting transgenic plant. It also includes a recombinant nucleic acid that is part of a hybrid nucleic acid encoding an additional polypeptide or peptide sequence.

The term "isolated" can further refer to a nucleic acid, nucleotide sequence, polypeptide, peptide or fragment that is substantially free of cellular material, viral material, and/or culture medium (e.g., when produced by recombinant DNA techniques), or chemical precursors or other chemicals (e.g., when chemically synthesized). Moreover, an "isolated fragment" is a fragment of a nucleic acid, nucleotide sequence or polypeptide that is not naturally occurring as a fragment and would not be found as such in the natural state. "Isolated" does not mean that the preparation is technically pure (homogeneous), but it is sufficiently pure to provide the polypeptide or nucleic acid in a form in which it can be used for the intended purpose.

In representative embodiments of the invention, an "isolated" nucleic acid, nucleotide sequence, and/or polypeptide is at least about 5%, 10%, 15%, 20%, 25%, 30%, 40%, 50%, 60%, 70%, 75%, 80%, 85%, 90%, 95%, 97%, 98%, 99% pure (w/w) or more. In other embodiments, an "isolated" nucleic acid, nucleotide sequence, and/or polypeptide indicates that at least about a 5-fold, 10-fold, 25-fold, 100-fold, 1000-fold, 10,000-fold, 100,000-fold or more enrichment of the nucleic acid (w/w) is achieved as compared with the starting material.

The terms "polypeptide," "protein," and "peptide" refer to a chain of covalently linked amino acids. In general, the term "peptide" can refer to shorter chains of amino acids (*e.g.,* 2-50 amino acids); however, all three terms overlap with respect to the length of the amino acid chain. As used herein, the terms "protein" and "polypeptide" are used interchangeably and encompass peptides, unless indicated otherwise. Polypeptides, proteins, and peptides may comprise naturally occurring amino acids, non-naturally occurring amino acids, or a combination of both. The polypeptides, proteins, and peptides may be isolated from sources (*e.g.,* cells or tissues) in which they naturally occur, produced recombinantly in cells *in vivo* or *in vitro* or in a test tube *in vitro,* or synthesized chemically. Such techniques are known to those skilled in the art. *See, e.g.,* Sambrook et al., Molecular Cloning: A Laboratory Manual 2nd Ed. (Cold Spring Harbor, NY, 1989); Ausubel et al. Current Protocols in Molecular Biology (Green Publishing Associates, Inc. and John Wiley & Sons, Inc., New York).

The term "fragment," as applied to a polypeptide, will be understood to mean an amino acid sequence of reduced length relative to a reference polypeptide or amino acid sequence and comprising, consisting essentially of, and/or consisting of an amino acid sequence of contiguous amino acids identical to the reference polypeptide or amino acid sequence. Such a polypeptide fragment according to the invention may be, where appropriate, included in a larger polypeptide of which it is a constituent. In some embodiments, such fragments can comprise, consist essentially of, and/or consist of peptides having a length of at least about 4, 6, 8, 10, 12, 15, 20, 25, 30, 35, 40, 45, 50, 75, 100, 150, 200, or more consecutive amino acids of a polypeptide or amino acid sequence according to the invention. A fragment of a polypeptide or protein can be produced by methods well known and routine in the art, for example, by enzymatic or other cleavage of naturally occurring peptides or polypeptides or by synthetic protocols that are well known.

A polypeptide fragment can be a biologically active fragment. A "biologically active fragment" or "active fragment" refers to a fragment that retains one or more of the biological activities of the reference polypeptide. Such fragments can be tested for biological activities according to methods described in the art, which are routine methods for testing activities of polypeptides, and/or according to any art-known and routine methods for identifying such activities. The production and testing to identify biologically active fragments of a polypeptide would be well within the scope of one of ordinary skill in the art and would be routine. Thus, the present invention further provides biologically active fragments of a polypeptide such as a polypeptide of interest and the polynucleotides encoding such biologically active polypeptide fragments.

The term "transgene" as used herein, refers to any nucleic acid sequence used in the transformation of a plant, animal, or other organism. Thus, a transgene can be a coding sequence, a non-coding sequence, a cDNA, a gene or fragment or portion thereof, a genomic sequence, a regulatory element and the like. A "transgenic" organism, such as a transgenic plant, transgenic microorganism, or transgenic animal, is an organism into which a transgene has been delivered or introduced and the transgene can be expressed in the transgenic organism to produce a product, the presence of which can impart an effect and/or a phenotype in the organism.

Different nucleic acids or polypeptides having homology are referred to herein as "homologues." The term homologue includes homologous sequences from the same and other species and orthologous sequences from the same and other species. "Homology" refers to the level of similarity between two or more nucleic acid and/or amino acid sequences in terms of percent of positional identity (i.e., sequence similarity or identity). Homology also refers to the concept of similar functional properties among different nucleic acids or proteins.

As used herein "sequence identity" refers to the extent to which two optimally aligned polynucleotide or polypeptide sequences are invariant throughout a window of alignment of components, *e.g*., nucleotides or amino acids. "Identity" can be readily calculated by known methods including, but not limited to, those described in: Computational Molecular Biology (Lesk, A. M., ed.) Oxford University Press, New York (1988); Biocomputing: Informatics and Genome Projects (Smith, D. W., ed.) Academic Press, New York (1993); Computer Analysis of Sequence Data, Part I (Griffin, A. M., and Griffin, H. G., eds.) Humana Press, New Jersey (1994); Sequence Analysis in Molecular Biology (von Heinje, G., ed.) Academic Press (1987); and Sequence Analysis Primer (Gribskov, M. and Devereux, J., eds.) Stockton Press, New York (1991).

An "identity fraction" for aligned segments of a test sequence and a reference sequence is the number of identical components which are shared by the two aligned sequences divided by the total number of components in reference sequence segment, *i.e.,* the entire reference sequence or a smaller defined part of the reference sequence. As used herein, the term "percent sequence identity" or "percent identity" refers to the percentage of identical nucleotides in a linear polynucleotide sequence of a reference ("query") polynucleotide molecule (or its complementary strand) as compared to a test ("subject") polynucleotide molecule (or its complementary strand) when the two sequences are optimally aligned (with appropriate nucleotide insertions, deletions, or gaps totaling less than 20 percent of the reference sequence over the window of comparison). In some embodiments, "percent identity" can refer to the percentage of identical amino acids in an amino acid sequence.

Optimal alignment of sequences for aligning a comparison window are well known to those skilled in the art and may be conducted by tools such as the local homology algorithm of Smith and Waterman, the homology alignment algorithm of Needleman and Wunsch, the search for similarity method of Pearson and Lipman, and optionally by computerized implementations of these algorithms such as GAP, BESTFIT, FASTA, and TFASTA available as part of the GCG® Wisconsin Package® (Accelrys Inc., Burlington, Mass.). An "identity fraction" for aligned segments of a test sequence and a reference sequence is the number of identical components which are shared by the two aligned sequences divided by the total number of components in the reference sequence segment, *i.e.,* the entire reference sequence or a smaller defined part of the reference sequence. Percent sequence identity is represented as the identity fraction multiplied by 100. The comparison of one or more polynucleotide sequences may be to a full-length polynucleotide sequence or a portion thereof, or to a longer polynucleotide sequence. For purposes of this invention "percent identity" may also be determined using BLASTX version 2.0 for translated nucleotide sequences and BLASTN version 2.0 for polynucleotide sequences.

The percent of sequence identity can be determined using the "Best Fit" or "Gap" program of the Sequence Analysis Software Package™ (Version 10; Genetics Computer Group, Inc., Madison, Wis.). "Gap" utilizes the algorithm of Needleman and Wunsch (Needleman and Wunsch, J Mol. Biol. 48:443-453, 1970) to find the alignment of two sequences that maximizes the number of matches and minimizes the number of gaps. "BestFit" performs an optimal alignment of the best segment of similarity between two sequences and inserts gaps to maximize the number of matches using the local homology algorithm of Smith and Waterman (Smith and Waterman, Adv. Appl. Math., 2:482-489, 1981, Smith et al., Nucleic Acids Res. 11:2205-2220, 1983).

Useful methods for determining sequence identity are also disclosed in Guide to Huge Computers (Martin J. Bishop, ed., Academic Press, San Diego (1994)), and Carillo, H., and Lipton, D., (Applied Math 48:1073(1988)). More particularly, preferred computer programs for determining sequence identity include but are not limited to the Basic Local Alignment Search Tool (BLAST) programs which are publicly available from National Center Biotechnology Information (NCBI) at the National Library of Medicine, National Institute of Health, Bethesda, Md. 20894; see BLAST Manual, Altschul et al., NCBI, NLM, NIH; (Altschul et al., J. Mol. Biol. 215:403-410 (1990)); version 2.0 or higher of BLAST programs allows the introduction of gaps (deletions and insertions) into alignments; for peptide sequence BLASTX can be used to determine sequence identity; and, for polynucleotide sequence BLASTN can be used to determine sequence identity.

Accordingly, the present invention further provides nucleotide sequences having significant sequence identity to the nucleotide sequences of the present invention. Significant sequence similarity or identity means at least 70%, 75%, 80%, 81%, 82%, 83%, 84%, 85%, 86%, 87%, 88%, 89%, 90%, 91%, 92%, 93%, 94%, 96%, 97%, 98%, 99% and/or 100% similarity or identity with another nucleotide sequence.

Additional aspects of the present invention provide methods of increasing resistance to a parasite in a plant, or part thereof, comprising: a) introducing into a plant cell a heterologous miRNA nucleotide sequence to produce a transgenic plant cell, wherein the heterologous miRNA nucleotide sequence is expressed in the transgenic plant cell; and b) regenerating a transgenic plant, or part thereof, from the transgenic plant cell of (a), wherein the transgenic plant, and/or part thereof, comprises in its genome the heterologous miRNA nucleotide sequence and has increased resistance to the plant parasite relative to a control plant, plant part, and/or plant cell that is not transformed with the heterologous miRNA nucleotide sequence.

In some embodiments of the present invention, the miRNA is miRNA164. In other embodiments of the present invention, the miRNA is another plant miRNA as described herein. In further embodiments, the miRNA is a miRNA that is identified as being expressed in roots as described herein.

Further aspects of the present invention provide methods of increasing resistance to a parasite in a plant, or part thereof, comprising: a) introducing into a plant cell a heterologous miR164 nucleotide sequence to produce a transgenic plant cell, wherein the heterologous miR164 nucleotide sequence is expressed in the transgenic plant cell; and b) regenerating a transgenic plant, or part thereof, from the transgenic plant cell of (a), wherein the transgenic plant, and/or part thereof, comprises in its genome the heterologous miR164 nucleotide sequence and has increased resistance to the plant parasite relative to a control plant, plant part, and/or plant cell that is not transformed with the heterologous miR164 nucleotide sequence.

Thus, in particular embodiments of the present invention methods of increasing resistance to soybean cyst nematode parasitism in a soybean plant, or part thereof, are provided comprising: a) introducing into a soybean plant cell a heterologous miR164 nucleotide sequence to produce a transgenic soybean plant cell, wherein the heterologous miR164nucleotide sequence is expressed in the transgenic plant cell; and b) regenerating a transgenic soybean plant, or part thereof, from the transgenic soybean plant cell of (a), wherein the transgenic soybean plant, or part thereof, comprises in its genome the heterologous miR164 nucleotide sequence and has increased resistance to soybean cyst nematode parasitism relative to a control soybean plant, soybean plant part, and/or soybean plant cell that is not transformed with the heterologous miR164 nucleotide sequence. A non-limiting example of a heterologous miR164 nucleotide sequence is the nucleotide sequence of **SEQ ID. NO:1**.

Thus, in some embodiments of the present invention, a method of increasing resistance to soybean cyst nematode parasitism in a soybean plant, and/or part thereof, is provided, the method comprising: a) introducing into a soybean plant cell a heterologous nucleotide sequence encoding the nucleotide sequence of **SEQ ID NO:1** to produce a transgenic soybean plant cell, wherein the nucleotide sequence of **SEQ ID NO:1** is expressed in the transgenic plant cell; and b) regenerating a transgenic soybean plant, or part thereof, from the transgenic soybean plant cell of (a), wherein the transgenic soybean plant, or part thereof, comprises in its genome the heterologous nucleotide sequence encoding the nucleotide sequence of **SEQ ID NO:1** and has increased resistance to soybean cyst nematode parasitism relative to a control soybean plant, soybean plant part, and/or soybean plant cell that is not transformed with the heterologous nucleotide sequence encoding the nucleotide sequence of **SEQ ID NO:1.**

As described above, in some embodiments of the present invention, the heterologous miRNA nucleotide sequence is overexpressed in the transgenic plant, plant part and/or plant cell, thereby producing a transgenic plant, plant part, and/or plant cell having increased resistance to a parasite relative to a control plant, plant part, and/or plant cell that is not transformed with the heterologous miRNA nucleotide sequence. In some embodiments of the present invention, the heterologous miRNA nucleotide sequence is miR164.

Thus, in some embodiments of the present invention, the heterologous miR164 nucleotide sequence is overexpressed in the transgenic plant, plant part and/or plant cell, thereby producing a transgenic plant, plant part, and/or plant cell having increased resistance to a parasite relative to a control plant, plant part, and/or plant cell that is not transformed with the heterologous miR164 nucleotide sequence.

In particular embodiments of the present invention, the heterologous miR164 nucleotide sequence is overexpressed in the transgenic soybean plant, soybean plant part, and/or soybean plant cell, thereby producing a transgenic soybean plant, soybean plant part, and/or soybean plant cell having increased resistance to soybean cyst nematode parasitism relative to a control soybean plant, soybean plant part, and/or soybean plant cell that is not transformed with the heterologous miR164 nucleotide sequence.

"Introducing," in the context of a nucleotide sequence of interest (e.g., miR164), means presenting the nucleotide sequence of interest to the plant, plant part, and/or plant cell in such a manner that the nucleotide sequence gains access to the interior of a cell. Where more than one nucleotide sequence is to be introduced these nucleotide sequences can be assembled as part of a single polynucleotide or nucleic acid construct, or as separate polynucleotide or nucleic acid constructs, and can be located on the same or different transformation vectors. Accordingly, these polynucleotides can be introduced into plant cells in a single transformation event, in separate transformation events, or, e.g., as part of a breeding protocol. Thus, the term "transformation" as used herein refers to the introduction of a heterologous nucleic acid into a cell. Transformation of a cell may be stable or transient.

"Transient transformation" in the context of a polynucleotide means that a polynucleotide is introduced into the cell and does not integrate into the genome of the cell.

By "stably introducing" or "stably introduced" in the context of a polynucleotide introduced into a cell is intended the introduced polynucleotide is stably incorporated into the genome of the cell, and thus the cell is stably transformed with the polynucleotide.

"Stable transformation" or "stably transformed" as used herein means that a nucleic acid is introduced into a cell and integrates into the genome of the cell. As such, the integrated nucleic acid is capable of being inherited by the progeny thereof, more particularly, by the progeny of multiple successive generations. "Genome" as used herein also includes the nuclear and the plastid genome, and therefore includes integration of the nucleic acid into, for example, the chloroplast genome. Stable transformation as used herein can also refer to a transgene that is maintained extrachromasomally, for example, as a minichromosome.

Transient transformation may be detected by, for example, an enzyme-linked immunosorbent assay (ELISA) or Western blot, which can detect the presence of a peptide or polypeptide encoded by one or more transgene introduced into an organism. Stable transformation of a cell can be detected by, for example, a Southern blot hybridization assay of genomic DNA of the cell with nucleic acid sequences which specifically hybridize with a nucleotide sequence of a transgene introduced into an organism (e.g., a plant). Stable transformation of a cell can be detected by, for example, a Northern blot hybridization assay of RNA of the cell with nucleic acid sequences which specifically hybridize with a nucleotide sequence of a transgene introduced into a plant or other organism. Stable transformation of a cell can also be detected by, *e.g.,* a polymerase chain reaction (PCR) or other amplification reactions as are well known in the art, employing specific primer sequences that hybridize with target sequence(s) of a transgene, resulting in amplification of the transgene sequence, which can be detected according to standard methods Transformation can also be detected by direct sequencing and/or hybridization protocols well known in the art.

A nucleic acid of this invention (e.g., miR164) can be introduced into a cell by any method known to those of skill in the art. In some embodiments of the present invention, transformation of a cell comprises nuclear transformation. In other embodiments, transformation of a cell comprises plastid transformation (e.g., chloroplast transformation).

Procedures for transforming plants are well known and routine in the art and are described throughout the literature. Non-limiting examples of methods for transformation of plants include transformation via bacterial-mediated nucleic acid delivery (*e.g*., via *Agrobacteria*), viral-mediated nucleic acid delivery, silicon carbide or nucleic acid whisker-mediated nucleic acid delivery, liposome mediated nucleic acid delivery, microinjection, microparticle bombardment, calcium-phosphate-mediated transformation, cyclodextrin-mediated transformation, electroporation, nanoparticle-mediated transformation,, sonication, infiltration, PEG-mediated nucleic acid uptake, as well as any other electrical, chemical, physical (mechanical) and/or biological mechanism that results in the introduction of nucleic acid into the plant cell, including any combination thereof. General guides to various plant transformation methods known in the art include Miki et al. ("Procedures for Introducing Foreign DNA into Plants" in Methods in Plant Molecular Biology and Biotechnology, Glick, B. R. and Thompson, J. E., Eds. (CRC Press, Inc., Boca Raton, 1993), pages 67-88) and Rakowoczy-Trojanowska (Cell. Mol. Biol. Lett. 7:849-858 (2002)).

Thus, in some particular embodiments, the introducing into a plant, plant part and/or plant cell is via bacterial-mediated transformation, particle bombardment transformation, calcium-phosphate-mediated transformation, cyclodextrin-mediated transformation, electroporation, liposome-mediated transformation, nanoparticle-mediated transformation, polymer-mediated transformation, virus-mediated nucleic acid delivery, whisker-mediated nucleic acid delivery, microinjection, sonication, infiltration, polyethyleneglycol-mediated transformation, any other electrical, chemical, physical and/or biological mechanism that results in the introduction of nucleic acid into the plant, plant part and/or cell thereof, or a combination thereof.

*Agrobacterium-mediated* transformation is a commonly used method for transforming plants, in particular, dicot plants, because of its high efficiency of transformation and because of its broad utility with many different species. *Agrobacterium-mediated* transformation typically involves transfer of the binary vector carrying the foreign DNA of interest to an appropriate *Agrobacterium* strain that may depend on the complement of *vir* genes carried by the host *Agrobacterium* strain either on a co-resident Ti plasmid or chromosomally (Uknes et al. (1993) Plant Cell 5:159-169). The transfer of the recombinant binary vector to *Agrobacterium* can be accomplished by a triparental mating procedure using *Escherichia coli* carrying the recombinant binary vector, a helper *E. coli* strain that carries a plasmid that is able to mobilize the recombinant binary vector to the target *Agrobacterium* strain. Alternatively, the recombinant binary vector can be transferred to *Agrobacterium* by nucleic acid transformation (Höfgen & Willmitzer (1988) Nucleic Acids Res. 16:9877).

Transformation of a plant by recombinant *Agrobacterium* usually involves cocultivation of the *Agrobacterium* with explants from the plant and follows methods well known in the art. Transformed tissue is regenerated on selection medium carrying an antibiotic or herbicide resistance marker between the binary plasmid T-DNA borders.

Another method for transforming plants, plant parts and plant cells involves propelling inert or biologically active particles at plant tissues and cells. *See, e.g.,* US Patent Nos. 4,945,050; 5,036,006 and 5,100,792. Generally, this method involves propelling inert or biologically active particles at the plant cells under conditions effective to penetrate the outer surface of the cell and afford incorporation within the interior thereof. When inert particles are utilized, the vector can be introduced into the cell by coating the particles with the vector containing the nucleic acid of interest. Alternatively, a cell or cells can be surrounded by the vector so that the vector is carried into the cell by the wake of the particle. Biologically active particles (e.g., dried yeast cells, dried bacterium or a bacteriophage, each containing one or more nucleic acids sought to be introduced) also can be propelled into plant tissue.

Thus, in particular embodiments of the present invention, a plant cell can be transformed by any method known in the art and as described herein and intact plants can be regenerated from these transformed cells using any of a variety of known techniques. Plant regeneration from plant cells, plant tissue culture and/or cultured protoplasts is described, for example, in Evans et al. (Handbook of Plant Cell Cultures, Vol. 1, MacMilan Publishing Co. New York (1983)); and Vasil I. R. (ed.) (Cell Culture and Somatic Cell Genetics of Plants, Acad. Press, Orlando, Vol. I (1984), and Vol. II (1986)). Methods of selecting for transformed transgenic plants, plant cells and/or plant tissue culture are routine in the art and can be employed in the methods of the invention provided herein.

Likewise, the genetic properties engineered into the transgenic seeds and plants, plant parts, and/or plant cells of the present invention described above can be passed on by sexual reproduction or vegetative growth and therefore can be maintained and propagated in progeny plants. Generally, maintenance and propagation make use of known agricultural methods developed to fit specific purposes such as harvesting, sowing or tilling.

A nucleotide sequence therefore can be introduced into the plant, plant part and/or plant cell in any number of ways that are well known in the art. The methods of the invention do not depend on a particular method for introducing one or more nucleotide sequences into a plant, only that they gain access to the interior of at least one cell of the plant. Where more than one nucleotide sequence is to be introduced, they can be assembled as part of a single nucleic acid construct, or as separate nucleic acid constructs, and can be located on the same or different nucleic acid constructs. Accordingly, the nucleotide sequences can be introduced into the cell of interest in a single transformation event, in separate transformation events, or, for example, in plants, as part of a breeding protocol.

Embodiments of the invention are directed to expression cassettes designed to express the nucleic acids of the present invention (e.g., miRNAs, miRNA164). As used herein, "expression cassette" means a nucleic acid molecule having at least a control sequence operably linked to a nucleotide sequence of interest (*e.g.,* a miRNA sequence; a miR164 sequence). In this manner, for example, plant promoters in operable interaction with the nucleotide sequences for the miRNAs of the invention are provided in expression cassettes for expression in a plant, plant part and/or plant cell.

As used herein, the term "promoter" refers to a region of a nucleotide sequence that incorporates the necessary signals for the efficient expression of a coding sequence. This may include sequences to which an RNA polymerase binds, but is not limited to such sequences and can include regions to which other regulatory proteins bind together with regions involved in the control of protein translation and can also include coding sequences.

Furthermore, a "promoter" of this invention is a promoter capable of initiating transcription in a cell of a plant. Such promoters include those that drive expression of a nucleotide sequence constitutively, those that drive expression when induced, and those that drive expression in a tissue- or developmentally-specific manner, as these various types of promoters are known in the art.

For purposes of the invention, the regulatory regions (i.e., promoters, transcriptional regulatory regions, and translational termination regions) can be native/analogous to the plant, plant part and/or plant cell and/or the regulatory regions can be native/analogous to the other regulatory regions. Alternatively, the regulatory regions may be heterologous to the plant (and/or plant part and/or plant cell) and/or to each other (i.e., the regulatory regions). Thus, for example, a promoter can be heterologous when it is operably linked to a polynucleotide from a species different from the species from which the polynucleotide was derived. Alternatively, a promoter can also be heterologous to a selected nucleotide sequence if the promoter is from the same/analogous species from which the polynucleotide is derived, but one or both (i.e., promoter and polynucleotide) are substantially modified from their original form and/or genomic locus, or the promoter is not the native promoter for the operably linked polynucleotide.

The choice of promoters to be used depends upon several factors, including, but not limited to, cell- or tissue-specific expression, desired expression level, efficiency, inducibility and selectability. For example, where expression in a specific tissue or organ is desired, a tissue-specific promoter can be used (e.g., a root specific promoter). In contrast, where expression in response to a stimulus is desired, an inducible promoter can be used. Where continuous expression is desired throughout the cells of a plant, a constitutive promoter can be used. It is a routine matter for one of skill in the art to modulate the expression of a nucleotide sequence by appropriately selecting and positioning promoters and other regulatory regions relative to that sequence.

Therefore, in some instances, constitutive promoters can be used. Examples of constitutive promoters include, but are not limited to, cestrum virus promoter (cmp) (U.S. Patent No. 7,166,770), the rice actin 1 promoter (Wang et al. (1992) Mol. Cell. Biol. 12:3399-3406; as well as US Patent No. 5,641,876), CaMV 35S promoter (Odell et al. (1985) Nature 313:810-812), CaMV 19S promoter (Lawton et al. (1987) Plant Mol. Biol. 9:315-324), nos promoter (Ebert et al. (1987) Proc. Natl. Acad. Sci USA 84:5745-5749), Adh promoter (Walker et al. (1987) Proc. Natl. Acad. Sci. USA 84:6624-6629), sucrose synthase promoter (Yang & Russell (1990) Proc. Natl. Acad. Sci. USA 87:4144-4148), and the ubiquitin promoter.

Moreover, tissue-specific regulated nucleic acids and/or promoters have been reported in plants. Thus, in some embodiments, tissue specific promoters can be used. Some reported tissue-specific nucleic acids include those encoding the seed storage proteins (such as β-conglycinin, cruciferin, napin and phaseolin), zein or oil body proteins (such as oleosin), or proteins involved in fatty acid biosynthesis (including acyl carrier protein, stearoyl-ACP desaturase and fatty acid desaturases (fad 2-1)), and other nucleic acids expressed during embryo development (such as Bce4, *see, e.g.,* Kridl et al. (1991) Seed Sci. Res. 1:209-219; as well as EP Patent No. 255378). Thus, the promoters associated with these tissue-specific nucleic acids can be used in the present invention. Additional examples of tissue-specific promoters include, but are not limited to, the root-specific promoters *RCc3* (Jeong et al. Plant Physiol. 153:185-197 (2010)) and RB7 (U.S. Patent No. 5459252), the lectin promoter (Lindstrom et al. (1990) Der. Genet. 11:160-167; and Vodkin (1983) Prog. Clin. Biol. Res. 138:87-98), corn alcohol dehydrogenase 1 promoter (Dennis et al. (1984) Nucleic Acids Res. 12:3983-4000), S-adenosyl-L-metluonine synthetase (SAMS) (Vander Mijnsbrugge et al. (1996) Plant and Cell Physiology, 37(8):1108-1115), corn light harvesting complex promoter (Bansal et al. (1992) Proc. Natl. Acad. Sci. USA 89:3654-3658), corn heat shock protein promoter (O'Dell et al. (1985) EMBO J. 5:451-458; and Rochester et al. (1986) EMBO J. 5:451-458), pea small subunit RuBP carboxylase promoter (Cashmore, "Nuclear genes encoding the small subunit of ribulose-1,5-bisphosphate carboxylase" 29-39 In: Genetic Engineering of Plants (Hollaender ed., Plenum Press 1983; and Poulsen et al (1986) Mol. Gen. Genet. 205:193-200), Ti plasmid mannopine synthase promoter (Langridge et al. (1989) Proc. Natl. Acad. Sci. USA 86:3219-3223), Ti plasmid nopaline synthase promoter (Langridge *et al.* (1989), *supra*), petunia chalcone isomerase promoter (van Tunen et al. (1988) EMBO J. 7:1257-1263), bean glycine rich protein 1 promoter *(*Keller et al. (1989) Genes Dev. 3:1639-1646), truncated CaMV 35S promoter (O'Dell et al. (1985) Nature 313:810-812), potato patatin promoter (Wenzler et al. (1989) Plant Mol. Biol, 13:347-354), root cell promoter (Yamamoto et al. (1990) Nucleic Acids Res. 18:7449), maize zein promoter (Kriz et al. (1987) Mol. Gen. Genet. 207:90-98; Langridge et al. (1983) Cell 34:1015-1022; Reina et al. (1990) Nucleic Acids Res. 18:6425; Reina et al. (1990) Nucleic Acids Res. 18:7449; and Wandelt et al. (1989) Nucleic Acids Res. 17:2354), globulin-1 promoter (Belanger et al. (1991) Genetics 129:863-872), α-tubulin cab promoter (Sullivan et al. (1989) Mol. Gen. Genet. 215:431-440), PEPCase promoter (Hudspeth & Grula (1989) Plant Mol. Biol. 12:579-589), R gene complex-associated promoters (Chandler et al. (1989) Plant Cell 1:1175-1183), and chalcone synthase promoters (Franken et al. (1991) EMBO J. 10:2605-2612). Particularly useful for seed-specific expression is the pea vicilin promoter (Czako et al. (1992) Mol. Gen. Genet. 235:33-40; as well as US Patent Nos. 5,625,136). Other useful promoters for expression in mature leaves are those that are switched on at the onset of senescence, such as the SAG promoter from *Arabidopsis* (Gan et al. (1995) Science 270:1986-1988). In addition, promoters functional in plastids can be used. Non-limiting examples of such promoters include the bacteriophage T3 gene 9 5' UTR and other promoters disclosed in U.S. Patent No. 7,579,516. Other promoters useful with the present invention, include but are not limited to the S-E9 small subunit RuBP carboxylase promoter and the Kunitz trypsin inhibitor gene promoter (Kti3).

In some instances, inducible promoters can be used. Examples of inducible promoters include, but are not limited to, tetracycline repressor system promoters, Lac repressor system promoters, copper-inducible system promoters, salicylate-inducible system promoters (*e.g*., the PR1a system), glucocorticoid-inducible promoters (Aoyama et al. (1997) Plant J. 11:605-612), and ecdysone-inducible system promoters. Other inducible promoters include ABA-and turgor-inducible promoters, the auxin-binding protein gene promoter *(*Schwob et al. (1993) Plant J. 4:423-432), the UDP glucose flavonoid glycosyl-transferase promoter (Ralston et al. (1988) Genetics 119:185-197), the MPI proteinase inhibitor promoter (Cordero et al. (1994) Plant J 6:141-150), and the glyceraldehyde-3-phosphate dehydrogenase promoter (Kohler et al. (1995) Plant Mol. Biol. 29:1293-1298; Martinez et al. (1989) J. Mol. Biol. 208:551-565; and Quigley et al. (1989) J. Mol. Evol. 29:412-421). Also included are the benzene sulphonamide-inducible (US Patent No. 5,364,780) and alcohol-inducible (Int'l Patent Application Publication Nos. WO 97/06269 and WO 97/06268) systems and glutathione S-transferase promoters. Likewise, one can use any of the inducible promoters described in Gatz (1996) Current Opinion Biotechnol. 7:168-172 and Gatz (1997) Annu. Rev. Plant Physiol. Plant Mol. Biol. 48:89-108.

In addition to the promoters described above, the expression cassette also can include other regulatory sequences. As used herein, "regulatory sequences" means nucleotide sequences located upstream (5' non-coding sequences), within or downstream (3' non-coding sequences) of a coding sequence, and which influence the transcription, RNA processing or stability, or translation of the associated coding sequence. Regulatory sequences include, but are not limited to, enhancers, introns, translation leader sequences and polyadenylation signal sequences.

A number of non-translated leader sequences derived from viruses also are known to enhance gene expression. Specifically, leader sequences from Tobacco Mosaic Virus (TMV, the "ω-sequence"), Maize Chlorotic Mottle Virus (MCMV) and Alfalfa Mosaic Virus (AMV) have been shown to be effective in enhancing expression (Gallie et al. (1987) Nucleic Acids Res. 15:8693-8711; and Skuzeski et al. (1990) Plant Mol. Biol. 15:65-79). Other leader sequences known in the art include, but are not limited to, picomavirus leaders such as an Encephalomyocarditis (EMCV) 5' noncoding region leader (Elroy-Stein et al. (1989) Proc. Natl. Acad. Sci. USA 86:6126-6130); potyvirus leaders such as a Tobacco Etch Virus (TEV) leader (Allison et al. (1986) Virology 154:9-20); Maize Dwarf Mosaic Virus (MDMV) leader (Allison *et al.* (1986), *supra*); human immunoglobulin heavy-chain binding protein (BiP) leader (Macejak & Samow (1991) Nature 353:90-94); untranslated leader from the coat protein mRNA of AMV (AMV RNA 4; Jobling & Gehrke (1987) Nature 325:622-625); tobacco mosaic TMV leader (Gallie et al. (1989) Molecular Biology of RNA 237-256); and MCMV leader (Lommel et al. (1991) Virology 81:382-385). *See also*, Della-Cioppa et al. (1987) Plant Physiol. 84:965-968.

The expression cassette also can optionally include a transcriptional and/or translational termination region (*i.e.,* termination region) that is functional in plants. A variety of transcriptional terminators are available for use in expression cassettes and are responsible for the termination of transcription beyond the transgene and correct mRNA polyadenylation. The termination region may be native to the transcriptional initiation region, may be native to the operably linked nucleotide sequence of interest, may be native to the plant host, or may be derived from another source (*i.e.,* foreign or heterologous to the promoter, the nucleotide sequence of interest, the plant host, or any combination thereof). Appropriate transcriptional terminators include, but are not limited to, the CAMV 35S terminator, the tml terminator, the nopaline synthase terminator and the pea rbcs E9 terminator. These can be used in both monocotyledons and dicotyledons. In addition, a coding sequence's native transcription terminator can be used.

A signal sequence can be operably linked to nucleic acids of the present invention (e.g., miR164) to direct the nucleotide sequence into a cellular compartment. In this manner, the expression cassette will comprise a nucleotide sequence encoding the miRNA operably linked to a nucleic acid sequence for the signal sequence. The signal sequence may be operably linked at the N- or C- terminus of the miRNA.

Regardless of the type of regulatory sequence(s) used, they can be operably linked to the nucleotide sequence of the miRNA. As used herein, "operably linked" means that elements of a nucleic acid construct such as an expression cassette are configured so as to perform their usual function. Thus, regulatory or control sequences (*e.g.,* promoters) operably linked to a nucleotide sequence of interest are capable of effecting expression of the nucleotide sequence of interest. The control sequences need not be contiguous with the nucleotide sequence of interest, so long as they function to direct the expression thereof. Thus, for example, intervening untranslated, yet transcribed, sequences can be present between a promoter and a coding sequence, and the promoter sequence can still be considered "operably linked" to the coding sequence. A nucleotide sequence of the present invention (i.e., a miRNA) can be operably linked to a regulatory sequence, thereby allowing its expression in a cell and/or subject.

The expression cassette also can include a nucleotide sequence for a selectable marker, which can be used to select a transformed plant, plant part or plant cell. As used herein, "selectable marker" means a nucleic acid that when expressed imparts a distinct phenotype to the plant, plant part or plant cell expressing the marker and thus allows such transformed plants, plant parts or plant cells to be distinguished from those that do not have the marker. Such a nucleic acid may encode either a selectable or screenable marker, depending on whether the marker confers a trait that can be selected for by chemical means, such as by using a selective agent (*e.g*., an antibiotic, herbicide, or the like), or on whether the marker is simply a trait that one can identify through observation or testing, such as by screening (*e.g*., the R-locus trait). Of course, many examples of suitable selectable markers are known in the art and can be used in the expression cassettes described herein.

Examples of selectable markers include, but are not limited to, a nucleic acid encoding *neo* or *nptII,* which confers resistance to kanamycin, G418, and the like (Potrykus et al. (1985) Mol. Gen. Genet. 199:183-188); a nucleic acid encoding *bar,* which confers resistance to phosphinothricin; a nucleic acid encoding an altered 5-enolpyruvylshikimate-3-phosphate (EPSP) synthase, which confers resistance to glyphosate (Hinchee et al. (1988) Biotech. 6:915-922); a nucleic acid encoding a nitrilase such as *bxn* from *Klebsiella ozaenae* that confers resistance to bromoxynil (Stalker et al. (1988) Science 242:419-423); a nucleic acid encoding an altered acetolactate synthase (ALS) that confers resistance to imidazolinone, sulfonylurea or other ALS-inhibiting chemicals (EP Patent Application No. 154204); a nucleic acid encoding a methotrexate-resistant dihydrofolate reductase (DHFR) (Thillet et al. (1988) J. Biol. Chem, 263:12500-12508); a nucleic acid encoding a dalapon dehalogenase that confers resistance to dalapon; a nucleic acid encoding a mannose-6-phosphate isomerase (also referred to as phosphomannose isomerase (PMI)) that confers an ability to metabolize mannose (US Patent Nos. 5,767,378 and 5,994,629); a nucleic acid encoding an altered anthranilate synthase that confers resistance to 5-methyl tryptophan; and/or a nucleic acid encoding *hph* that confers resistance to hygromycin. One of skill in the art is capable of choosing a suitable selectable marker for use in an expression cassette.

Additional selectable markers include, but are not limited to, a nucleic acid encoding β-glucuronidase or *uidA* (GUS) that encodes an enzyme for which various chromogenic substrates are known; an R-locus nucleic acid that encodes a product that regulates the production of anthocyanin pigments (red color) in plant tissues (Dellaporta et al., "Molecular cloning of the maize R-nj allele by transposon-tagging with Ac" 263-282 In: Chromosome Structure and Function: Impact of New Concepts, 18th Stadler Genetics Symposium (Gustafson & Appels eds., Plenum Press 1988)); a nucleic acid encoding β-lactamase, an enzyme for which various chromogenic substrates are known (*e.g*., PADAC, a chromogenic cephalosporin) (Sutcliffe (1978) Proc. Natl. Acad. Sci. USA 75:3737-3741); a nucleic acid encoding *xylE* that encodes a catechol dioxygenase (Zukowsky et al. (1983) Proc. Natl, Acad. Sci. USA 80:1101-1105); a nucleic acid encoding tyrosinase, an enzyme capable of oxidizing tyrosine to DOPA and dopaquinone, which in turn condenses to form melanin (Katz et al. (1983) J. Gen. Microbiol. 129:2703-2714); a nucleic acid encoding β-galactosidase, an enzyme for which there are chromogenic substrates; a nucleic acid encoding luciferase (lux) that allows for bioluminescence detection (Ow et al. (1986) Science 234:856-859); a nucleic acid encoding aequorin which may be employed in calcium-sensitive bioluminescence detection (Prasher et al. (1985) Biochem. Biophys. Res. Comm. 126:1259-1268); or a nucleic acid encoding green fluorescent protein *(*Niedz et al. (1995) Plant Cell Reports 14:403-406). One of skill in the art is capable of choosing a suitable selectable marker for use in an expression cassette.

An expression cassette of the present invention also can include nucleotide sequences for coding for other desired traits. Such sequences can be stacked with any combination of nucleotide sequences to create plants, plant parts or plant cells having the desired phenotype. Stacked combinations can be created by any method including, but not limited to, cross breeding plants by any conventional methodology, or by genetic transformation. If stacked by genetically transforming the plants, the nucleotide sequences of interest can be combined at any time and in any order. For example, a transgenic plant comprising one or more desired traits can be used as the target to introduce further traits by subsequent transformation. The additional nucleotide sequences can be introduced simultaneously in a co-transformation protocol with nucleotide sequences for the heterologous miR164 provided by any combination of expression cassettes. For example, if two nucleotide sequences will be introduced, they can be incorporated in separate cassettes (trans) or can be incorporated on the same cassette (cis). Expression of the nucleotide sequences can be driven by the same promoter or by different promoters. It is further recognized that nucleotide sequences can be stacked at a desired genomic location using a site-specific recombination system. *See, e.g.,* Int'l Patent Application Publication Nos. WO 99/25821; WO 99/25854; WO 99/25840; WO 99/25855 and WO 99/25853.

The expression cassette also can include a coding sequence for one or more polypeptides for agronomic traits that primarily are of benefit to a seed company, grower or grain processor, for example, resistance to bacterial pathogens, fungal resistance, herbicide resistance, insect resistance, nematode resistance and virus resistance. *See, e.g.,* US Patent Nos. 5,304,730; 5,495,071; 5,569,823; 6,329,504 and 6,337,431. The trait also can be one that increases plant vigor or yield (including traits that allow a plant to grow at different temperatures, soil conditions and levels of sunlight and precipitation), or one that allows identification of a plant exhibiting a trait of interest (*e.g*., a selectable marker, seed coat color, *etc.*). Various traits of interest, as well as methods for introducing these traits into a plant, are described, for example, in US Patent Nos. 4,761,373; 4,769,061; 4,810,648; 4,940,835; 4,975,374; 5,013,659; 5,162,602; 5,276,268; 5,304,730; 5,495,071; 5,554,798; 5,561,236; 5,569,823; 5,767,366; 5,879,903, 5,928,937; 6,084,155; 6,329,504 and 6,337,431; as well as US Patent Application Publication No. 2001/0016956. *See also,* on the World Wide Web at lifesci.sussex.ac.uk/home/Neil_Crickmore/Bt/.

Numerous nucleotide sequences are known to enhance expression from within a transcriptional unit, and these sequences can be used in conjunction with the nucleotide sequence for the miRNA of the present invention (e.g., miR164) to increase its expression in transgenic plants. For example, introns of the maize Adhl gene and Intron 1 have been shown to enhance gene expression. *See, e.g.,* Callis et al. (1987) Genes Develop. 1:1183-1200.

The nucleotide sequence for the miRNA can be cloned into the expression cassette, and the resulting expression cassetted can be introduced/transformed into, for example, a plant, plant part and/or plant cell.

In addition to expression cassettes, numerous plant transformation vectors are well known to one of skill in the art, and the nucleotide sequences described herein can be used in connection with any such vectors. The selection of a vector will depend upon the preferred transformation technique and the target species for transformation. For certain target species, different antibiotic or herbicide selection markers may be preferred as discussed herein.

The term "vector" refers to a composition for transferring, delivering or introducing a nucleic acid (or nucleic acids) into a cell. A vector comprises a nucleic acid comprising the nucleotide sequence to be transferred, delivered or introduced. In some embodiments, a vector of this invention can be a viral vector, which can comprise, *e.g*., a viral capsid and/or other materials for facilitating entry of the nucleic acid into a cell and/or replication of the nucleic acid of the vector in the cell (*e.g.*, reverse transcriptase or other enzymes which are packaged within the capsid, or as part of the capsid). The viral vector can be an infectious virus particle that delivers nucleic acid into a cell following infection of the cell by the virus particle.

The present invention further provides a method of reducing soybean cyst nematode cyst formation in a plant, plant part, and/or plant cell, comprising: introducing into the plant, plant part, and/or plant cell a heterologous miRNA nucleotide sequence, wherein the heterologous miRNA nucleotide sequence is expressed in the plant, and/or part thereof, thereby reducing soybean cyst nematode cyst formation in a plant, plant part, and/or plant cell relative to a control plant, plant part, and/or plant cell that is not transformed with the heterologous miRNA nucleotide sequence. In some embodiments of the present invention, the heterologous miRNA nucleotide sequence is miR164.

Thus, the present invention further provides a method of reducing soybean cyst nematode cyst formation in a plant, plant part, and/or plant cell, comprising: introducing into the plant, plant part, and/or plant cell a heterologous miR164 nucleotide sequence, wherein the heterologous miR164 nucleotide sequence is expressed in the plant, and/or part thereof, thereby reducing soybean cyst nematode cyst formation in the plant, plant part, and/or plant cell relative to a control plant, plant part, and/or plant cell that is not transformed with the heterologous miR164 nucleotide sequence.

The present invention further provides a method of reducing soybean cyst nematode cyst formation in a soybean plant, soybean plant part, and/or soybean plant cell, comprising: introducing into the soybean plant, soybean plant part, and/or soybean plant cell a heterologous miR164 nucleotide sequence, wherein the heterologous miR164 nucleotide sequence is expressed in the plant, and/or part thereof, thereby reducing soybean cyst nematode cyst formation in the soybean plant, soybean plant part, and/or soybean plant cell relative to a control soybean plant, soybean plant part, and/or soybean plant cell that is not transformed with the heterologous miR164 nucleotide sequence. A non-limiting example of a heterologous miR164 nucleotide sequence is the nucleotide sequence of **SEQ ID. NO:1.**

Thus, in some embodiments of the present invention, a method of reducing soybean cyst nematode formation in a soybean plant, and/or part thereof, is provided, the method comprising introducing into the soybean plant, soybean plant part, and/or soybean plant cell, a heterologous nucleotide sequence encoding the nucleotide sequence of **SEQ ID NO:1,** wherein the heterologous nucleotide sequence is expressed in the plant, plant part and/or plant cell, thereby producing a transgenic soybean plant, soybean plant part, and/or soybean plant cell having reduced soybean cyst nematode formation relative to a control soybean plant, soybean plant part, and/or soybean plant cell that is not transformed with the heterologous nucleotide sequence encoding the nucleotide sequence of **SEQ ID NO:1.**

Further embodiments of the present invention provide methods for regenerating a transgenic plant from the transgenic plant part or plant cell, wherein the regenerated transgenic plant comprises in its genome a heterologous miRNA nucleotide sequence and has increased resistance to soybean cyst nematode parasitism and or reduced soybean cyst nematode cyst formation in a plant relative to a control (as defined herein). Plants can be regenerated from transformed plant parts, plant tissues, and/or plant cells using any of a variety of known techniques as described above.

Still further embodiments of the present invention provide methods for regenerating a transgenic plant from the transgenic plant part or plant cell, wherein the regenerated transgenic plant comprises in its genome a heterologous miR164 nucleotide sequence and has increased resistance to soybean cyst nematode parasitism and or reduced soybean cyst nematode cyst formation in a plant relative to a control (as defined herein).

Other embodiments of the present invention provide methods for regenerating a transgenic plant from the transgenic plant part or plant cell, wherein the regenerated transgenic plant comprises in its genome the heterologous miR164 nucleotide sequence and has increased resistance to soybean cyst nematode parasitism and/or reduced soybean cyst nematode cyst formation in a plant relative to a control (as defined herein).

Additional embodiments of the present invention provide methods for regenerating a transgenic soybean plant from the transgenic soybean plant part or soybean plant cell, wherein the regenerated transgenic soybean plant comprises in its genome the heterologous miR164 nucleotide sequence and has increased resistance to soybean cyst nematode parasitism and or reduced soybean cyst nematode cyst formation in a soybean plant relative to a control (as defined herein). Plants can be regenerated from transformed plant parts, plant tissues, and/or plant cells using any of a variety of known techniques as described above.

In some embodiments, the present invention provides methods of reducing soybean cyst nematode cyst formation in a soybean plant, and/or part thereof, comprising a) introducing into a soybean plant cell a heterologous miR164 nucleotide sequence to produce a transgenic plant cell, wherein the heterologous miR164 nucleotide sequence is expressed in the plant cell; and b) regenerating a transgenic soybean plant, and/or part thereof, from the transgenic soybean plant cell of (a), wherein the transgenic soybean plant, and/or part thereof, comprises in its genome the heterologous miR164 nucleotide sequence and has reduced formation of soybean cyst nematode cysts relative to a control soybean plant, soybean plant part, and/or soybean plant cell that is not transformed with the heterologous miR164 nucleotide sequence. A non-limiting example of a heterologous miR164 nucleotide sequence is the nucleotide sequence of **SEQ ID. NO:1**.

Thus, in some embodiments of the present invention, a method of reducing soybean cyst nematode cyst formation in a soybean plant, or part thereof, is provided, the method comprising a) introducing into a soybean plant cell a heterologous nucleotide sequence encoding the nucleotide sequence of **SEQ ID NO:1,** wherein the nucleotide sequence of **SEQ ID NO:1** is expressed in the plant cell; and b) regenerating a transgenic soybean plant, and/or part thereof, from the soybean plant cell of (a), wherein the transgenic soybean plant, or part thereof, comprises in its genome the heterologous nucleotide sequence encoding the nucleotide sequence of **SEQ ID NO:1** and has reduced soybean cyst nematode cyst formation relative to a control soybean plant, soybean plant part, and/or soybean cell that is not transformed with the heterologous nucleotide sequence encoding the nucleotide sequence of **SEQ ID NO:1.**

As previously discussed, in some embodiments of the present invention, the heterologous miR164 nucleotide sequence is overexpressed in the soybean plant, soybean plant part, and/or soybean cell into which the heterologous miRNA is introduced. Thus, in some embodiments, the present invention provides a method of reducing soybean cyst nematode cyst formation in a soybean plant, soybean plant part, and/or soybean plant cell, comprising: introducing into the soybean plant, soybean plant part, and/or soybean plant cell a heterologous miR164 nucleotide sequence, wherein the heterologous miR164 nucleotide sequence (e.g., **SEQ ID NO:1**) is overexpressed in the plant, and/or part thereof, thereby reducing soybean cyst nematode cyst formation in a soybean plant, soybean plant part, and/or soybean plant cell relative to a control soybean plant, soybean plant part, and/or soybean plant cell that is not transformed with the heterologous miR164 nucleotide sequence.

Additionally, the present invention provides methods of reducing soybean cyst nematode cyst formation in a soybean plant, and/or part thereof, comprising a) introducing into a soybean plant cell a heterologous miR164 nucleotide sequence to produce a transgenic soybean plant cell, wherein the heterologous miR164 nucleotide sequence (e.g., **SEQ ID NO:1)** is overexpressed in the plant cell; and b) regenerating a transgenic soybean plant, and/or part thereof, from the transgenic soybean plant cell of (a), wherein the transgenic soybean plant, and/or part thereof, comprises in its genome the heterologous miR164 nucleotide sequence and has reduced formation of soybean cyst nematode cysts relative to a control soybean plant, soybean plant part, and/or soybean plant cell that is not transformed with the heterologous miR164 nucleotide sequence.

The terms "reduce," "reduced," "reducing," "reduction," "diminish," and "decrease" (and grammatical variations thereof), as used herein, describe a decrease in the soybean cyst nematode cyst formation on a plant (e.g., soybean) by the introduction of a miRNA of the present invention into the plant, thereby producing a transgenic plant having decreased or reduced cyst formation on the transgenic plant. This decrease in cyst formation can be observed, by comparing the number of cysts formed on the plant transformed with the heterologous miR164 nucleotide sequence to the number formed on a soybean plant that is not transformed with the heterologous miR164 nucleotide sequence.

In an additional aspect, the present invention further provides methods of obtaining a progeny plant derived from a transgenic plant of the invention, wherein said progeny plant comprises in its genome a heterologous miRNA nucleotide sequence and has increased resistance to a plant parasite relative to a control plant not transformed with the heterologous miRNA nucleotide sequence.

In a further aspect, the present invention provides methods of obtaining a progeny plant derived from a transgenic plant of the invention, wherein said progeny plant comprises in its genome the heterologous miR164 nucleotide sequence and has increased resistance to a plant parasite relative to a control plant not transformed with the heterologous miR164 nucleotide sequence. In particular embodiments, the present invention provides methods of obtaining a progeny soybean plant derived from the transgenic soybean plant, wherein said progeny plant comprises in its genome the heterologous miR164 nucleotide sequence and has increased resistance to soybean cyst nematode parasitism relative to a control soybean plant as defined herein. In still further embodiments, the present invention provides methods of obtaining a progeny soybean plant derived from the transgenic soybean plant, wherein said progeny plant comprises in its genome a heterologous nucleotide sequence encoding the nucleotide sequence of **SEQ ID NO:1** and has increased resistance to soybean cyst nematode parasitism relative to a control soybean plant as defined herein

In other embodiments of the present invention, methods of obtaining a progeny soybean plant derived from the transgenic soybean plant are provided, wherein said progeny plant comprises in its genome a heterologous miRNA nucleotide sequence and has reduced formation of soybean cyst nematode cysts relative to a control soybean plant that is not transformed with the heterologous miRNA nucleotide sequence.

In still other embodiments of the present invention, methods of obtaining a progeny soybean plant derived from the transgenic soybean plant are provided, wherein said progeny plant comprises in its genome the heterologous miR164 nucleotide sequence and has reduced formation of soybean cyst nematode cysts relative to a control soybean plant that is not transformed with the heterologous miR164 nucleotide sequence. Thus, in some embodiments, the present invention provides methods of obtaining a progeny soybean plant derived from the transgenic soybean plant, wherein said progeny plant comprises in its genome the heterologous miR164 nucleotide sequence and has reduced formation of soybean cyst nematode cysts relative to a control as defined herein. In still further embodiments, the present invention provides methods of obtaining a progeny soybean plant derived from the transgenic soybean plant, wherein said progeny plant comprises in its genome a heterologous nucleotide sequence encoding the nucleotide sequence of **SEQ ID NO:1** and has reduced formation of soybean cyst nematode cysts relative to a control as defined herein

As discussed above, the heterologous miRNA nucleotide sequence (e.g., miR164) can be present in a nucleic acid construct such as an expression cassette and/or vector. When present in a nucleic acid construct, the heterologous miRNA nucleotide sequence can be operably associated with a promoter (e.g., upstream of the heterologous miRNA nucleotide sequence) as described above. A nucleic acid construct comprising the heterologous miRNA nucleotide sequence can further comprise a NOS terminator operably associated with the heterologous miRNA nucleotide sequence (e.g., downstream from the heterologous miRNA nucleotide sequence). Thus, in particular embodiments, the present invention provides a nucleic acid construct comprising the heterologous miRNA nucleotide sequence operably associated with a promoter and a NOS terminator. In other embodiments, the present invention provides a nucleic acid construct comprising the heterologous miR164 nucleotide sequence operably associated with a promoter and a NOS terminator.

In other embodiments, the present invention provides plants, plant parts and/or plant cells having increased resistance to a plant parasite produced by the methods of the present invention. Thus, in some embodiments, the present invention provides plants, plant parts and/or plant cells having increased resistance to soybean cyst nematode parasitism produced by the methods of the present invention. In still other embodiments, the present invention provides soybean plants, soybean plant parts and/or soybean plant cells having increased resistance to soybean cyst nematode parasitism produced by the methods of the present invention.

Further aspects of the present invention provide plants, plant parts and/or plant cells having reduced formation of soybean cyst nematode cysts produced by the methods of the present invention. In still further aspects, the present invention provides soybean plants, soybean plant parts and/or soybean plant cells having reduced formation of soybean cyst nematode cysts produced by the methods of the present invention.

In some embodiments, the present invention provides transgenic plants grown from transgenic seed comprising a heterologous miRNA nucleotide sequence (e.g., miRNA 164), as well as progeny generated from plants including inbred and hybrid plant lines made by crossing a transgene plant grown from the transgenic seed to a second plant not grown from the same transgenic seed. Additionally provided is soybean seed produced by the soybean plant of the present invention, wherein the seed comprises the heterologous miRNA nucleotide sequence. In some embodiments, the present invention provides transgenic plants grown from the transgenic seed comprising the heterologous miRNA nucleotide sequence as well as progeny generated from plants including inbred and hybrid plant lines made by crossing a transgene plant grown from the transgenic seed to a second plant not grown from the same transgenic seed. Further embodiments of the present invention provide soybean seed meal produced from the soybean seed of the present invention.

The present invention will now be described with reference to the following examples. It should be appreciated that these examples are for the purpose of illustrating aspects of the present invention, and does not limit the scope of the invention as defined by the claims.

### EXAMPLES

### Example 1. Preparation of expression cassettes.

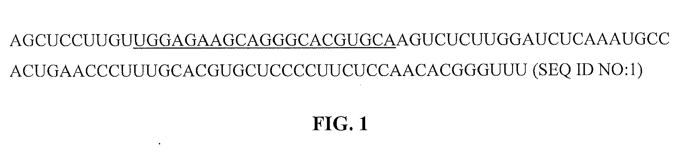

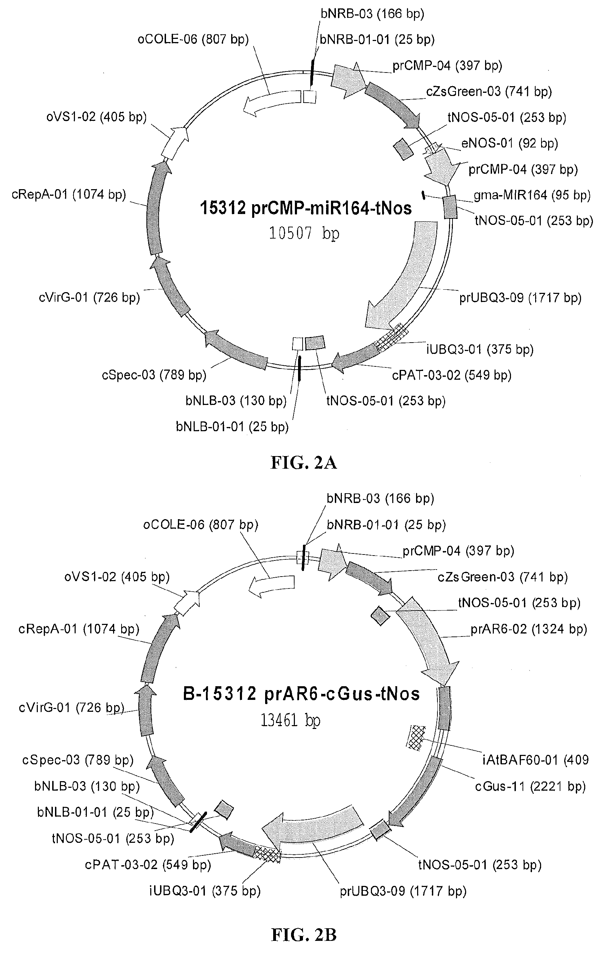

miR164 (gma-miR164; **Fig. 1****; SEQ ID NO:1**) was cloned into a prCMP:MIR164:tNOS expression cassette. The expression cassette with the miR164 was then cloned into a binary vector to create the vector pKS033 (a.k.a. 15312 prCMP-MIR164-tNOS; shown in **Fig. 2**).

The pKS033 vector was then transformed into *Agrobacterium rhizogenes* Strain K599. As control, binary vector pKS001 (a.k.a. B-15312-prAR6-cGUS-tNOS) with prAR6:GUS:tNOS cassette was transformed into the *A. rhizogenes* K599 strain. In the case of the control, the expression cassette does not include miR164.

### Example 2. Induction and selection of transgenic soybean hairy root events harboring the prCMP:MIR164:tNOS expression cassette and the prAR6:GUS:tNOS cassette.

Narayanan et al. (Crop Science 39:1680-1686 (1999)) indicated that SCN resistance phenotypes in the whole plant were preserved in transgenic hairy roots, therefore transgenic hairy root system is useful for evaluating candidate SCN resistance genes. Accordingly, the soybean hairy root system was used to evaluate resistance to SCN. Soybean cultivar Williams 82 was used as the gerplasm for the hairy root transformation. Seeds of soybean seeds were germinated on 1% agar containing 0.5% sucrose in petri dishes at 27 °C for 5 days. The cotyledons were then cut off the seedlings, and the wounded surface was inoculated with cultures of the Agrobacterium carrying pKS033 and pKS001. The cotyledons were placed on 1 % agar for 6 days and then transferred onto selection media. In about two weeks, independent transgenic hair root events induced from the cotyledons were harvested and transferred onto culture media, and cultured in the darkness at 27 °C.

### Example 3. Evaluation of cyst formation in soybean hairy root system.

### Evaluation in hairs roots comprising the prCMP:MIR164:tNOS expression cassette.

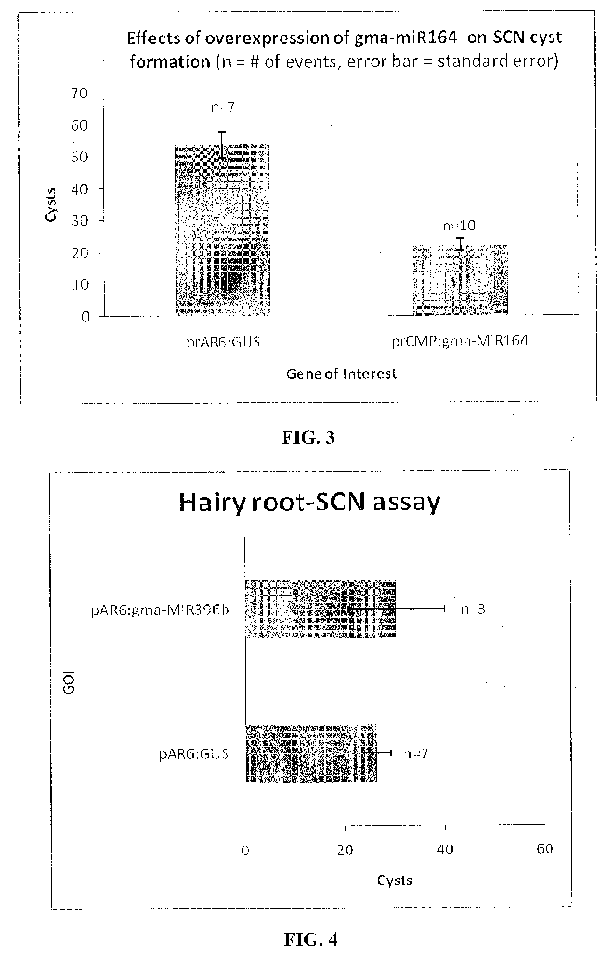

Two weeks after transfer onto the culture plates, the transformed hairy roots were inoculated with surface-sterilized J2 stage soybean cyst nematodes (SCN J2) and the roots were grown in darkness at 27 °C, which allowed cyst formation on the hairy root events. One month after nematode inoculation, the number of cysts was determined for both the roots comprising the prCMP:MIR164:tNOS expression cassette and the roots comprising the prAR6:GUS:tNOS cassette. As shown in **Fig. 3****,** the average cyst number in the roots overexpressing miR164 was found to be significantly lower than that for the control plants that express the GUS gene (i.e., no miR164) (p<0.01), based on ANOVA single factor analysis.

### Evaluation in hairy roots comprising the prAR6:gma-MIR396b expression cassette

In a similar experiment, another soybean miRNA, gma-miR396a, was over-expressed. As shown in Fig. 4, the overexpression of miR396b did not reduce the cyst developed on the transgenic hairy roots as compared to the control.

### Example 4. Transformation of soybean with the binary vector 18963.

miR164 (gma-miR164; **Fig. 1****; SEQ ID NO:1**) was cloned into a prCMP:MIR164:tNOS expression cassette **(****Fig. 2A****).** The expression cassette with the miR164 was then cloned into a binary vector to create the vector 18963. The 18963 vector was then transformed into *A. tumefaciens* Strain EHA101.

Transformation of soybean to produce transgenic soybean plants is accomplished using immature seed targets of variety Williams 82 via *A. tumefaciens-mediated* transformation using explant materials and media recipes as described in Hwang et al. (WO08112044) and Que et al. (WO08112267) except where noted below. Using this method, genetic elements within the left and right border regions of the transformation plasmid are efficiently transferred and integrated into the genome of the plant cell, while genetic elements outside these border regions are generally not transferred. Maturing soybean pods are harvested from greenhouse grown plants, sterilized with diluted bleach solution and rinsed with sterile water. Immature seeds are then excised from seed pods and rinsed with sterile water briefly. Explants are prepared from sterilized immature seeds as described in Hwang et al. (WO08112044) and infected with *A. tumefaciens* strain EHA101 harboring the transformation binary vector 18963 and allowed to incubate for an additional 30 to 240 minutes. Excess *A. tumefaciens* suspension is then removed by aspiration and the explants are moved to plates containing a non-selective co-culture medium. The explants are co-cultured with the remaining *A. tumefaciens* at 23°C for 4 days in the dark and then transferred to recovery and regeneration medium supplemented with an antibiotics mixture consisting of ticarcillin (75 mg/L), cefotaxime (75 mg/L) and vancomycin (75 mg/l) where they are incubated in the dark for seven days. The explants are then transferred to regeneration medium containing hygromycin B (3 to 6 mg/L) and a mixture of antibiotics consisting of ticarcillin (75 mg/L), cefotaxime (75 mg/L) and vancomycin (75 mg/l) to inhibit and kill *A. tumefaciens.* Shoot regeneration and elongation is carried out in elongation media containing 2-4 mg/L of hygromycin B. The hygromycin phosphor-transferease (HPT) gene is used as a selectable marker during the transformation process. Regenerated plantlets are transplanted to soil as described (WO08112267) and tested for the presence of both HPT and CMP promoter sequences by TaqMan PCR analysis (Ingham et al., 2001). This screen allows for the selection of transgenic events that carry the T-DNA and are free of vector backbone DNA. Plants positive for HPT gene and CMP sequences and negative for the spectinomycin (spec) gene were transferred to the greenhouse for analysis of miRNA expression seed setting.

Plants transformed with prAR6:GUS:tNOS **(****Fig. 2B****)** expression cassette are used as the control.

### Example 5. Evaluation of cyst formation in the transformed plants.

Plants transformed with the prCMP:MIR164:tNOS expression cassette are inoculated with J2 stage soybean cyst nematodes (SCN J2). Briefly, 3-week old seedling of the transgenic T1 generation soybean seedlings grown in pots are inoculated with SCN J2 suspension at the level of 3000 J2 per plant. One month after nematode inoculation, the number of cysts is determined for both the transgenic plants comprising the prCMP:MIR164:tNOS expression cassette and for the null segregants from the same T0 parents.

Although the foregoing invention has been described in some detail by way of illustration and example for purposes of clarity of understanding, it will be obvious that certain changes and modifications may be practiced within the scope of the list of the foregoing embodiments and the appended claims.

### Disclosed Aspects:

Disclosed herein is a method of producing a transgenic soybean plant, soybean plant part, or soybean plant cell having increased resistance to a nematode plant parasite, comprising:
introducing into the soybean plant, soybean plant part, or soybean plant cell, a heterologous miRNA nucleotide sequence, wherein the heterologous miRNA nucleotide sequence is expressed in the soybean plant, soybean plant part, or soybean plant cell, thereby producing a transgenic soybean plant, soybean plant part, or soybean plant cell having increased resistance to a nematode plant parasite relative to a control soybean plant, soybean plant part, or soybean plant cell.

Further, disclosed is a method of increasing resistance to a nematode plant parasite in a soybean plant, or part thereof, particularly a soybean cyst nematode, comprising: a) introducing into a soybean plant cell a heterologous miRNA nucleotide sequence to produce a transgenic soybean plant cell, wherein the heterologous miRNA nucleotide sequence is expressed in the soybean transgenic plant cell; and b) regenerating a soybean transgenic soybean plant, or part thereof, from the soybean transgenic plant cell of (a), wherein the soybean transgenic plant, and/or part thereof, comprises in its genome the heterologous miRNA nucleotide sequence and has increased resistance to the nematode plant parasite relative to a control soybean plant, and/or part thereof, that is not transformed with the heterologous miRNA nucleotide sequence.

Also disclosed herein is a method of producing a transgenic plant, plant part, or plant cell having increased resistance to a plant parasite, comprising:
introducing into the plant, plant part, or plant cell, a heterologous miR164 nucleotide sequence, wherein the heterologous miR164 nucleotide sequence is expressed in the plant, plant part or plant cell, thereby producing a transgenic plant, plant part, or plant cell having increased resistance to a plant parasite relative to a control plant, plant part, or plant cell.

Particularly, said method further comprises regenerating a transgenic plant from the transgenic plant part or plant cell, wherein the regenerated transgenic plant comprises in its genome the heterologous miR164 nucleotide sequence and has increased resistance to a plant parasite relative to a control plant.

More particularly, said method further comprises obtaining a progeny plant derived from the transgenic plant, wherein said progeny plant comprises in its genome the heterologous miR164 nucleotide sequence and has increased resistance to a plant parasite relative to a control plant.

Further disclosed herein is a method of producing a transgenic soybean plant, soybean plant part, or soybean plant cell having increased resistance to soybean cyst nematode parasitism, comprising:
introducing into the soybean plant, soybean plant part, or soybean plant cell, a heterologous miR164 nucleotide sequence, wherein the heterologous miR164 nucleotide sequence is expressed in the soybean plant, soybean plant part or soybean plant cell, thereby producing a transgenic soybean plant, soybean plant part, or soybean plant cell having increased resistance to soybean cyst nematode parasitism relative to a control soybean plant, soybean plant part, or soybean plant cell.

Particularly, said method further comprises obtaining a progeny soybean plant derived from the transgenic soybean plant, wherein said progeny plant comprises in its genome the heterologous miR164 nucleotide sequence and has increased resistance to soybean cyst nematode parasitism relative to a control soybean plant.

Particularly, said method further comprises regenerating a transgenic soybean plant from the transgenic soybean plant part or soybean plant cell, wherein the regenerated transgenic soybean plant comprises in its genome the heterologous miR164 nucleotide sequence and has increased resistance to soybean cyst nematode parasitism relative to a control soybean plant and, optionally, obtaining a progeny soybean plant derived from the regenerated transgenic soybean plant, wherein said progeny plant comprises in its genome the heterologous miR164 nucleotide sequence and has increased resistance to soybean cyst nematode parasitism relative to a control soybean plant.

Also disclosed is a method of increasing resistance to soybean cyst nematode parasitism in a soybean plant, or part thereof, comprising:
a) introducing into a soybean plant cell a heterologous miR164 nucleotide sequence to produce a transgenic soybean plant cell, wherein the heterologous miR164nucleotide sequence is expressed in the transgenic soybean plant cell; and
b) regenerating a transgenic soybean plant, or part thereof, from the transgenic soybean plant cell of (a), wherein the transgenic soybean plant, or part thereof, comprises in its genome the heterologous miR164 nucleotide sequence and has increased resistance to soybean cyst nematode parasitism relative to a control soybean plant, or part thereof.

Particularly, said method further comprises obtaining a progeny soybean plant derived from the transgenic soybean plant, wherein said progeny plant comprises in its genome the heterologous miR164 nucleotide sequence and has increased resistance to soybean cyst nematode parasitism relative to a control soybean plant.

Further disclosed is a method of reducing soybean cyst nematode cyst formation in a soybean plant, soybean plant part, or soybean plant cell, comprising:
introducing into the soybean plant, soybean plant part, or soybean plant cell a heterologous miR164 nucleotide sequence, wherein the heterologous miR164 nucleotide sequence is expressed in the soybean plant, soybean plant part, or soybean plant cell, thereby reducing soybean cyst nematode cyst formation in the soybean plant, soybean plant part, or soybean plant cell relative to a control soybean plant, soybean plant part, or soybean plant cell.

Particularly, said method further comprises obtaining a progeny soybean plant derived from the transgenic soybean plant, wherein said progeny plant comprises in its genome the heterologous miR164 nucleotide sequence and has reduced soybean cyst nematode formation relative to a control soybean plant.

Particularly, said method further comprises regenerating a transgenic soybean plant from the transgenic soybean plant part or soybean cell, wherein the regenerated transgenic soybean plant comprises in its genome the heterologous miR164 nucleotide sequence and has reduced soybean cyst nematode formation relative to a control soybean plant, and, optionally, obtaining a progeny soybean plant derived from the transgenic soybean plant, wherein said progeny plant comprises in its genome the heterologous miR164 nucleotide sequence and has reduced soybean cyst nematode formation relative to a control soybean plant.

In another aspect a method of reducing soybean cyst nematode cyst formation in a soybean plant, or part thereof, is disclosed comprising
a) introducing into a soybean plant cell a heterologous miR164 nucleotide sequence to produce a transgenic soybean plant cell, wherein the heterologous miR164 nucleotide sequence is expressed in the soybean plant cell; and
b) regenerating a transgenic soybean plant, or part thereof, from the transgenic soybean plant cell of (a), wherein the transgenic soybean plant, or part thereof, comprises in its genome the heterologous miR164 nucleotide sequence and has reduced formation of soybean cyst nematode cysts relative to a control soybean plant, or part thereof.

In particular, said method further comprises obtaining a progeny soybean plant derived from the transgenic soybean plant, wherein said progeny plant comprises in its genome the heterologous miR164 nucleotide sequence and has reduced soybean cyst nematode formation relative to a control soybean plant

In particular, the heterologous miR164 nucleotide sequence used in the method as disclosed herein is present in a nucleic acid construct, particularly said heterologous miR164 nucleotide sequence is operably associated with a promoter.

Further, within the method disclosed herein, the heterologous miR164 nucleotide sequence is introduced into -a soybean plant cell via bacterial-mediated transformation, particle bombardment transformation, calcium-phosphate-mediated transformation, cyclodextrin-mediated transformation, electroporation, liposome-mediated transformation, nanoparticle-mediated transformation, polymer-mediated transformation, virus-mediated nucleic acid delivery, whisker-mediated nucleic acid delivery, microinjection, sonication, infiltration, polyethyleneglycol-mediated transformation, any other electrical, chemical, physical or biological mechanism that results in the introduction of nucleic acid into the plant, plant part or cell thereof, or a combination thereof.

Further disclosed herein is a plant, plant part or plant cell having increased resistance to a plant parasite, particularly a soybean cyst nematode parasite, produced by the method disclosed herein.

Further, disclosed is a soybean plant, soybean plant part or soybean plant cell having reduced formation of soybean cyst nematode cysts produced by the method disclosed herein.

Also disclosed is a soybean seed which is produced by the soybean plant disclosed herein, wherein the seed comprises the heterologous miR164 nucleotide sequence.

Further disclosed is a soybean seed meal, which is produced from the soybean seed disclosed herein.

## Claims

1. A method of producing a transgenic plant, plant part, or plant cell having increased resistance to a nematode plant parasite, comprising:
introducing into the plant, plant part, or plant cell, a heterologous miRNA nucleotide sequence, wherein the heterologous miRNA nucleotide sequence comprises a miR164 nucleotide sequence that is at least 75% identical to SEQ ID NO:1, wherein the heterologous miRNA nucleotide sequence is expressed in the plant, plant part, or plant cell, thereby producing a transgenic plant, plant part, or plant cell having increased resistance to a nematode plant parasite relative to a control plant, plant part, or plant cell.

2. The method of claim 1, wherein the plant, plant part, or plant cell is a soybean plant, soybean plant part, or soybean plant cell.

3. The method of claim 2, wherein the nematode plant parasite is soybean cyst nematode.

4. The method of claim 3, wherein the transgenic soybean plant, soybean plant part, or soybean plant cell has reduced soybean cyst nematode cyst formation relative to a control soybean plant, soybean plant part, or soybean plant cell.

5. The method of claim 1, further comprising regenerating a transgenic plant from the transgenic plant part or plant cell, wherein the regenerated transgenic plant comprises in its genome the heterologous miRNA nucleotide sequence and has increased resistance to the nematode plant parasite relative to a control plant.

6. The method of any of claims 2 to 4, further comprising regenerating a transgenic soybean plant from the transgenic soybean plant part or plant cell, wherein the regenerated transgenic soybean plant comprises in its genome the heterologous miRNA- nucleotide sequence and has increased resistance to the nematode plant parasite relative to a control soybean plant, and/or part thereof and/or has reduced soybean cyst nematode cyst formation relative to a control soybean plant, and/or part thereof.

7. The method of any of claims 1 to 6, further comprising obtaining a progeny soybean plant derived from the transgenic soybean plant, wherein said progeny plant comprises in its genome the heterologous miRNA nucleotide sequence and has increased resistance to nematode parasitism and/or reduced soybean cyst nematode cyst formation relative to a control soybean plant

8. The method of any one of claims 1 to 7, wherein the heterologous miR164 nucleotide sequence is present in a nucleic acid construct. 8

9. The method of claim 8, wherein the heterologous miR164 nucleotide sequence is operably associated with a promoter.

10. The method of any of claims 1 to 9, wherein the introducing is via bacterial-mediated transformation, particle bombardment transformation, calcium-phosphate-mediated transformation, cyclodextrin-mediated transformation, electroporation, liposome-mediated transformation, nanoparticle-mediated transformation, polymer-mediated transformation, virus-mediated nucleic acid delivery, whisker-mediated nucleic acid delivery, microinjection, sonication, infiltration, polyethyleneglycol-mediated transformation, any other electrical, chemical, physical or biological mechanism that results in the introduction of nucleic acid into the plant, plant part or cell thereof, or a combination thereof.

11. A soybean plant, soybean plant part or soybean plant cell having increased resistance to nematode parasitism, soybean cyst nematode parasitism, and/or reduced formation of soybean cyst nematode cysts, wherein said soybean plant, soybean plant part or soybean plant cell comprises a heterologous miRNA nucleotide sequence comprising a miR164 nucleotide sequence that is at least 75% identical to SEQ ID NO:1, wherein the heterologous miRNA nucleotide sequence is expressed in the soybean plant, soybean plant part, or soybean plant cell, thereby producing a transgenic soybean plant, soybean plant part, or soybean plant cell having increased resistance to a nematode plant parasite relative to a control plant, plant part, or plant cell.

12. A soybean seed produced by the soybean plant of claim 11, wherein the seed comprises in its genome the heterologous miRNA nucleotide sequence as defined in claim 11.

13. The soybean seed of claim 12, wherein the heterologous miR164 nucleotide sequence comprises the nucleotide sequence of SEQ ID NO:1.

14. A soybean seed meal produced from the soybean seed of claim 12 or claim 13, wherein the seed meal comprises the heterologous miRNA nucleotide sequence.

## Patentansprüche

1. Verfahren zur Erzeugung einer transgenen Pflanze, eines transgenen Pflanzenteils oder einer transgene Pflanzenzelle mit erhöhter Resistenz gegen einen pflanzenparasitären Nematoden, das Folgendes umfasst:
Einführen einer heterologen miRNA-Nukleotidsequenz in die Pflanze, den Pflanzenteil oder die Pflanzenzelle, wobei die heterologe miRNA-Nukleotidsequenz eine miR164-Nukleotidsequenz mit mindestens 75% Identität zu SEQ ID NO: 1 umfasst, wobei die heterologe miRNA-Nukleotidsequenz in der Pflanze, dem Pflanzenteil oder der Pflanzenzelle exprimiert wird, wodurch eine transgene Pflanze, ein transgener Pflanzenteil oder eine transgene Pflanzenzelle mit einer im Vergleich zu einer Kontrollpflanze, einem Kontrollpflanzenteil oder einer Kontrollpflanzenzelle erhöhten Resistenz gegen einen pflanzenparasitären Nematoden erzeugt wird.

2. Verfahren nach Anspruch 1, wobei es sich bei der Pflanze, dem Pflanzenteil oder der Pflanzenzelle um eine Sojabohnenpflanze, einen Sojabohnenpflanzenteil oder eine Sojabohnenpflanzenzelle handelt.

3. Verfahren nach Anspruch 2, wobei es sich bei dem pflanzenparasitären Nematoden um einen Sojabohnenzystennematoden handelt.

4. Verfahren nach Anspruch 3, wobei die transgene Sojabohnenpflanze, der transgene Sojabohnenpflanzenteil oder die transgene Sojabohnenpflanzenzelle eine im Vergleich zu einer Kontrollsojabohnenpflanze, einem Kontrollsojabohnenpflanzenteil oder einer Kontrollsojabohnenpflanzenzelle verringerte Sojabohnenzystennematodenzysten-bildung aufweist.

5. Verfahren nach Anspruch 1, das weiterhin das Regenerieren einer transgenen Pflanze, aus dem transgenen Pflanzenteil oder der transgenen Pflanzenzelle umfasst, wobei die regenerierte transgene Pflanze in ihrem Genom die heterologe miRNA-Nukleotidsequenz umfasst und eine im Vergleich zu einer Kontrollpflanze erhöhte Resistenz gegen den pflanzenparasitären Nematoden aufweist.

6. Verfahren nach einem der Ansprüche 2 bis 4, das weiterhin das Regenerieren einer transgenen Sojabahnenpflanze aus dem transgenen Sojabohnenpflanzenteil oder der transgenen Sojabohnenpflanzenzelle umfasst, wobei die regenerierte transgene Soja-bohnenpflanze in ihrem Genom die heterologe miRNA-Nukleotidsequenz umfasst und im Vergleich zu einer Kontrollsojabohnenpflanze und/oder einem Teil davon eine erhöhte Resistenz gegen den pflanzenparasitären Nematoden aufweist und/oder eine im Vergleich zu einer Kontrollsojabohnenpflanze und/oder einem Teil davon verringerte Sojabohnenzystennematodenzystenbildung aufweist.

7. Verfahren nach einem der Ansprüche 1 bis 6, das weiterhin das Erhalten einer Nachkommensojabohnenpflanze umfasst, die sich von der transgenen Sojabohnenpflanze ableitet, wobei die Nachkommenpflanze in ihrem Genom die heterologe miRNA-Nukleotidsequenz umfasst und im Vergleich zu einer Kontrollsojabohnenpflanze eine erhöhte Resistenz gegen Nematodenparasitismus und/oder eine verringerte Sojabohnenzystennematodenzystenbildung aufweist.

8. Verfahren nach einem der Ansprüche 1 bis 7, wobei die heterologe miR164-Nukleotidsequenz in einem Nukleinsäurekonstrukt vorliegt.

9. Verfahren nach Anspruch 8, wobei die heterologe miR164-Nukleotidsequenz operativ mit einem Promoter verknüpft ist.

10. Verfahren nach einem der Ansprüche 1 bis 9, wobei das Einführen erfolgt durch bakterienvermittelte Transformation, Transformation, mittels Teilchenbeschuss, kalziumphosphatvermittelter Transformation, cyclodextrinvermittelter Transformation, Elektroporation, liposomenvermittelter Transformation, nanopartikelvermittelter Transformation, polymervermittelter Transformation, virusvermittelter Nukleinsäureabgabe, whiskervermittelter Nukleinsäureabgabe, Mikroinjektion, Ultraschallbehandlung, Infiltration, polyethylenglykolvermittelter Transformation, einen beliebigen anderen elektrischen, chemischen, physikalischen oder biologischen Mechanismus, der in der Einführung von Nukleinsäure in die Pflanze , den Pflanzenteil oder die Zelle davon resultiert, oder eine Kombination davon.

11. Sojabohnenpflanze, Sojabohnenpflanzenteil oder Sojabohnenpflanzenzelle mit erhöhter Resistenz gegen Nematodenparasitismus, Sojabohnenzystennematodenparasitismus und/oder verringerter Bildung von Sojabohnenzystennematodenzysten, wobei die Sojabohnenpflanze, der Sojabohnenpflanzenteil oder die Sojabohnenpflanzenzelle eine heterologe miRNA-Nukleotidsequenz umfassend eine miR164-Nukleotidsequenz mit mindestens 75% Identität zu SEQ ID NO: 1 umfasst, wobei die heterologe miRNA-Nukleotidsequenz in der Sojabohnenpflanze, dem Sojabohnenpflanzenteil oder der Sojabohnenpflanzenzelle exprimiert wird, wodurch eine transgene Sojabohnenpflanze, ein transgener Sojabohnenpflanzenteil oder eine transgene Sojabohnenpflanzenzelle mit einer im Vergleich zu einer Kontrollpflanze, einem Kontrollpflanzenteil oder einer Kontrollpflanzenzelle erhöhten Resistenz gegen einen pflanzenparasitären Nematoden erzeugt wird.

12. Sojabohnensamen, der von der Sojabohnenpflanze nach Anspruch 11 erzeugt wird, wobei der Samen die heterologe miRNA-Nukleotidsequenz wie in Anspruch 11 definiert in seinem Genom umfasst.

13. Sojabohnensamen nach Anspruch 12, wobei die heterologe miR164-Nukleotidsequenz die Nukleotidsequenz von SEQ ID NO: 1 umfasst.

14. Sojabohnensamenschrot, hergestellt aus dem Sojabohnensamen nach Anspruch 12 oder Anspruch 13, wobei der Samenschrot die heterologe miRNA-Nukleotidsequenz umfasst.

## Revendications

1. Méthode de production d'une plante, d'une partie de plante ou d'une cellule végétale, transgénique, ayant une résistance accrue vis-à-vis d'un parasite de plante de type nématode, comprenant :
l'introduction dans la plante, la partie de plante ou la cellule végétale, d'une séquence nucléotidique de miARN hétérologue, où la séquence nucléotidique de miARN hétérologue comprend une séquence nucléotidique de miR164 qui possède une identité d'au moins 75% avec SEQ ID n° 1, où la séquence nucléotidique de miARN hétérologue est exprimée dans la plante, la partie de plante ou la cellule végétale, produisant ainsi une plante, une partie de plante ou une cellule végétale, transgénique, ayant une résistance accrue vis-à-vis d'un parasite de plante de type nématode par rapport à une plante, une partie de plante ou une cellule végétale, témoin.

2. Méthode selon la revendication 1, **caractérisée en ce que** la plante, la partie de plante ou la cellule végétale est une plante de soja, une partie de plante de soja ou une cellule végétale de soja.

3. Méthode selon la revendication 2, **caractérisée en ce que** le parasite de plante de type nématode est le nématode à kystes du soja.

4. Méthode selon la revendication 3, **caractérisée en ce que** la plante de soja, la partie de plante de soja ou la cellule végétale de soja, transgénique, possède une formation réduite de kystes par le nématode à kystes du soja, par rapport à une plante de soja, une partie de plante de soja ou une cellule végétale de soja, témoin.

5. Méthode selon la revendication 1, comprenant en outre la régénération d'une plante transgénique à partir de la partie de plante ou la cellule végétale transgénique, **caractérisée en ce que** la plante transgénique régénérée comprend dans son génome la séquence nucléotidique de miARN hétérologue et possède une résistance accrue vis-à-vis du parasite de plante de type nématode par rapport à une plante témoin.

6. Méthode selon l'une quelconque des revendications 2 à 4, comprenant en outre la régénération d'une plante de soja transgénique à partir de la partie de plante ou la cellule végétale de soja transgénique, **caractérisée en ce que** la plante de soja transgénique régénérée comprend dans son génome la séquence nucléotidique de miARN hétérologue et possède une résistance accrue vis-à-vis du parasite de plante de type nématode par rapport à une plante de soja témoin, et/ou une partie de celle-ci et/ou possède une formation réduite de kystes par le nématode à kystes du soja, par rapport à une plante de soja témoin et/ou une partie de celle-ci.

7. Méthode selon l'une quelconque des revendications 1 à 6, comprenant en outre l'obtention d'une descendance de plante de soja dérivée de la plante de soja transgénique, **caractérisée en ce que** ladite descendance de plante comprend dans son génome la séquence nucléotidique de miARN hétérologue et possède une résistance accrue vis-à-vis d'un parasitisme de type nématode et/ou une formation réduite de kystes par le nématode à kystes du soja, par rapport à une plante de soja témoin.

8. Méthode selon l'une quelconque des revendications 1 à 7, **caractérisée en ce que** la séquence nucléotidique de miR164 hétérologue est présente dans une construction d'acide nucléique.

9. Méthode selon la revendication 8, **caractérisée en ce que** la séquence nucléotidique de miR164 hétérologue est associée de manière opérable avec un promoteur.

10. Méthode selon l'une quelconque des revendications 1 à 9, **caractérisée en ce que** l'introduction se fait via transformation médiée par des bactéries, transformation par bombardement de particules, transformation médiée par du phosphate de calcium, transformation médiée par de la cyclodextrine, électroporation, transformation médiée par des liposomes, transformation médiée par des nanoparticules, transformation médiée par des polymères, administration d'acide nucléique médiée par des virus, administration d'acide nucléique médiée par des trichites, micro-injection, traitement par ultrasons, infiltration, transformation méditée par du polyéthylène glycol, tout autre mécanisme électrique, chimique, physique ou biologique qui entraîne l'introduction d'acide nucléique dans la plante, la partie de plante ou une cellule de celle-ci, ou leur combinaison.

11. Plante de soja, partie de plante de soja ou cellule végétale de soja, ayant une résistance accrue vis-à-vis du parasitisme de type nématode, du parasitisme de type nématode à kystes du soja, et/ou ayant une formation réduite de kystes par le nématode à kystes du soja, **caractérisée en ce que** ladite plante de soja, ladite partie de plante de soja ou ladite cellule végétale de soja comprend une séquence nucléotidique de miARN hétérologue comprenant une séquence nucléotidique de miR164 qui possède une identité d'au moins 75% avec SEQ ID n° 1, où la séquence nucléotidique de miARN hétérologue est exprimée dans la plante de soja, la partie de plante de soja ou la cellule végétale de soja, produisant ainsi une plante de soja, une partie de plante de soja ou une cellule végétale de soja, transgénique, ayant une résistance accrue vis-à-vis d'un parasite de plante de type nématode par rapport à une plante, une partie de plante ou une cellule végétale, témoin.

12. Graine de soja produite par la plante de soja selon la revendication 11, **caractérisée en ce que** la graine comprend dans son génome la séquence nucléotidique de miARN hétérologue telle que définie selon la revendication 11.

13. Graine de soja selon la revendication 12, **caractérisée en ce que** la séquence nucléotidique de miR164 hétérologue comprend la séquence nucléotidique de SEQ ID n° 1.

14. Farine de graines de soja produite à partir des graines de soja selon la revendication 12 ou la revendication 13, **caractérisée en ce que** la farine de graines comprend la séquence nucléotidique de miARN hétérologue.


### SEQUENCE LISTING

<110> Syngenta Participations AG
   Huang, Xiang
   Seguin, Katie
<120> OVEREXPRESSION OF PLANT miRNAS FOR PARASITE CONTROL
<130> 9207-21WO
<150> US 61/408,088
   <151> 2010-10-29
<160> 1
<170> PatentIn version 3.5
<210> 1
   <211> 95
   <212> RNA
   <213> Glycine max
<400> 1
   agcuccuugu uggagaagca gggcacgugc aagucucuug gaucucaaau gccacugaac 60
   ccuuugcacg ugcuccccuu cuccaacacg gguuu 95